# EUROPEAN PATENT APPLICATION

(11) **EP 2 924 045 A1**
(43) Date of publication of application: **30.09.2015**
(21) Application number: 14161424.8
(22) Date of filing: 25.03.2014
(51) Int. Cl.: C07F 15/06, A61P 35/00, A61K 31/295, A61K 31/517

(54) **New agents for the treatment of cancer**

(71) Applicant: Medizinische Universität Wien, 1090 Vienna (AT); Universität Wien, 1010 Wien (AT)
(72) Inventor: HEFFETER, Petra, 1030 Vienna (AT); KOWOL, Christian, 1140 Vienna (AT); BERGER, Walter, 1140 Vienna (AT); KEPPLER, Bernhard, 1291 Gaweinstal (AT)
(74) Representative: Sonn & Partner Patentanwälte

(57) **Abstract**

The invention relates to compounds comprising a Co³⁺ ion and a tyrosine kinase inhibitor moiety, wherein the tyrosine kinase inhibitor moiety comprises a Co³⁺-chelating moiety and wherein the tyrosine kinase inhibitor moiety is a 4-anilinoquinazoline tyrosine kinase inhibitor moiety. The 4-anilinoquinazoline tyrosine kinase inhibitor moiety has the general formula B, wherein R₁, R₂, R₃ and m are as defined herein. The compounds are useful in the treatment of hyperproliferative diseases, such as cancer.

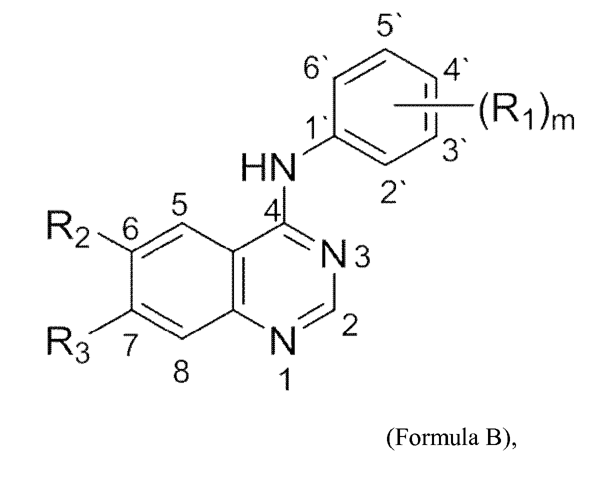

## Description

The present invention discloses new agents for the treatment of cancer.

Cancer is one of the leading causes of death worldwide, accounting for about 13% of all deaths (i.e. 7.6 million) in 2008. Overall, there is a broad spectrum of diverse malignant neoplasms with lung cancer being number one cause of cancer-related deaths (1.3 million worldwide), followed by stomach (736 000), liver (695 000) and colorectal cancer (608 000). Basically, there are three therapeutic options for the treatment of malignancies: surgery, irradiation and systemic chemotherapy including classic cytostatic drugs as well as new targeted approaches using small molecules, antibodies, etc. While at early stages cancer can be cured by surgery, diagnosis of late stages characterised by cancer dissemination causes the need for systemic treatment options. Unfortunately, these strategies are neither in all nor even in the majority of cases curative. Thus, systemic cancer treatment options are frequently combined in highly complex schemes. Often chemotherapy or radiation therapy are given before (neoadjuvant) or after (adjuvant) local surgery. Moreover, diverse drug combination protocols exist.

There is a huge diversity in clinically used chemotherapeutic drugs including several antibiotics (e.g. daunomycin, adriamycin), plant toxins (e.g. vincristine, paclitaxel), antimetabolites (e.g. 5-fluorouracil, cytarabine), as well as several metal compounds (e.g. cisplatin, oxaliplatin). Despite their advantages and numerous applications, a major disadvantage of cytotoxic agents is their limited cancer specificity resulting in frequent occurrence of severe side effects. This is based on the fact that all of their targets are also expressed within normal dividing cells and especially in those with high proliferation rates, like hair follicle, bone marrow and skin cells as well as gastrointestinal mucosa and thus are also damaged by the classical cytotoxic drugs. With the aim to overcome these limitations, in the last decade novel drugs have been developed, which should selectively inhibit oncogenic targets (such as growth factor receptors) and pathways. During the last years the development of targeted therapies led to distinct improvement of cancer therapy. However, although their mode of action differs from classical chemotherapeutics, still the occurrence of drug resistance and severe side effects are major limitations to successful treatment, especially at the advanced stage.

One of the most successful classes of targeted compounds are the so-called tyrosine kinase inhibitors, among which many are directed against oncogenic growth factor receptors.

Thus, it is the object of the present invention to provide new and efficient compounds for the treatment of cancer with reduced toxicity and/or enhanced tumor selectivity, e.g. compared to already established EGFR inhibitors. Since there are a number of already successful compounds for the treatment of cancer, there is also a desire to further develop and improve such already known and efficient structures. It is therefore another object to create derivatives of already known efficient cancer drugs with improved properties.

The present invention provides new targeted EGFR inhibitors, specifically for use as antitumor drugs. EGFR is a receptor tyrosine kinase (RTK) which belongs, along with HER2/ErbB2, HER3/ErbB3 and HER4/ErbB4, to the HER family. These membrane-located proteins all consist of an extracellular ligand-binding domain, a hydrophobic membrane-spanning region and an intracellular tyrosine kinase domain which catalyses the transfer of a γ-phosphate group of adenosine triphosphate (ATP) to the hydroxyl group of a tyrosine residue within the protein leading to autophosphorylation of the receptor and activation of its downstream signaling.

In contrast to non-malignant, healthy cells, where the initiation of proliferation-promoting signaling pathways is tightly controlled, one of the most important characteristics of cancer cells is their ability to evade growth inhibitory signals and sustain chronic proliferation as well as their self-sufficiency in growth signals. Thus, upregulation of growth factor-induced pathways (e.g. mediated by HER family members) is frequently linked to malignant transformations. Especially EGFR and HER2 have been found to play a role in cancer progression. Additionally to cell proliferation and survival, HER-induced downstream signaling pathways have been shown to be important for tumor invasion and metastasis.

There are various mechanisms leading to deregulation of EGFR-controlled pathways:
1. Overexpression of growth factors
2. Overexpression of the EGFR (either as a consequence of gene amplification, or due to increased mRNA production), leading to an increased probability of receptor dimerisation and therefore signal transduction. This has been detected to varying degrees in a wide range of solid tumors, particularly those of head and neck, kidney, lung, and breast (the tumor type percentage of tumors expressing EGFR is: head and neck: 80-100; renal (50-90); lung 40-80; breast 14-91; colon 25-77; ovarian 35-70; prostate 39-47; glioma 40-63; pancreas 30-50; bladder 31-48).
3. Co-expression of EGFR with one of its ligands, enhancing the efficiency of autocrine stimulation of cell growth.
4. Overexpression of HER2, which often occurs in breast cancer. Notably, HER2 does not have any ligands, but is able to form stable heterodimers with, for example, EGFR.
5. Mutations in the EGFR gene, including different deletions (e.g. ΔE746-A750) in the extracellular or intracellular domain, which promote constitutive activation of the receptor without the need of any ligands. Such mutations have been detected in various human cancers, including lung carcinomas and glioblastomas, and have been associated with clinical benefit from EGFR-targeted therapy.

The present invention relates to small-molecule tyrosine kinase inhibitors (TKIs). Several classes of EGFR TKIs have been identified during the last two decades, including 4-anilinoquinazolines, 4-anilino-quinoline-3-carbonitriles, 1*H*-pyrazolo[3,4-*d*]pyrimidines, 7*H-*pyrrolo[2,3-*d*]pyrimidines, pyrrolotriazines, 4-amino-6-arylamino-pyrimidines and thienopyrimidines. Among them, 4-anilinoquinazolines are the most potent and selective inhibitors as well as the most investigated so far. Furthermore, until today only EGFR TKIs with this core structure have been approved for clinical use. Thus, this structural fragment seems to be important for EGFR binding. Since the discovery of the EGFR inhibitory activity of 4-anilinoquinazolines, a huge amount of studies was performed to get detailed insights into their structure-activity relationship (SAR).

In addition to the pyrimidine ring, which is important and in some compounds crucial for EGFR binding, also the nature of the linking group between the 4-position of the quinazoline and the phenyl group, which occupies a hydrophobic pocket of the catalytic domain, is important for the inhibitory activity. Here, a secondary amino group as linker was found to be the most effective moiety, because N-methylation dramatically lowered the activity of the TKIs. Regarding substitution patterns, it can be concluded that substituents on the aniline ring should be lipophilic, electron-withdrawing groups, like Cl or Br and preferentially localized at the 3'or 4'-position, whereas on the quinazoline core electron-donating substituents at the 6- and/or 7-position are preferred. In contrast, 5-substitution at the quinazoline moiety reduces the potency due to a conformational effect and 8-substitution based on decreased N1 basicity. Introduction of a substituent at 2-position has also shown to be disfavoured.

Based on these key parameters, a vast number of 4-anilinoquinazoline derivatives has been synthesized and some (pre)clinically developed. Among them, gefitinib and erlotinib were the first TKIs to be approved by the U.S. Food and Drug Administration (FDA) for clinical use (gefitinib 2003; erlotinib 2004). In Europe, gefitinib is marketed under the brand name Iressa^{®} by AstraZenecaAB since 2009 and is indicated for the treatment of patients with locally advanced or metastatic NSCLC with activating mutations of the EGFR. Erlotinib/Tarceva^{®}, which is sold by Hoffmann-La Roche Ltd., has been approved in 2005 for first-line treatment of patients with locally advanced or metastatic NSCLC with EGFR-activating mutations, as monotherapy in treatment-refractory, advanced NSCLC and in combination with gemcitabine, a nucleoside analogue, for the treatment of patients with metastatic pancreatic cancer.

The third EGFR TKI in clinical use is lapatinib, a dual inhibitor of EGFR and HER2. This drug has been approved by the European Commission in 2008 for the treatment of patients with breast cancer, whose tumors overexpress HER2 and in combination with capecitabine for advanced or metastatic disease with progression following prior therapy.

EGFR mutations have been shown to play a critical role in the sensitivity to targeted therapy using TKIs. Reversible EGFR inhibitors, such as erlotinib and gefitinib, were discovered to be mainly effective in patients carrying various somatic mutations that hyperactivate the EGFR kinase. These mutations are for example the deletion ΔE746-A750 or the point mutation L858R, which comprise each approximately 40-45% of EGFR mutations in NSCLC. Based on the results of several clinical studies, which demonstrated good response of patients with activating mutations to gefitinib treatment in contrast to nearly no response of patients with wild-type EGFR, approval restrictions have been made. Thus, gefitinib, which was first indicated for general treatment of locally advanced or metastatic NSCLC, is now only approved for patients with activating EGFR mutations. The correlation between such mutations and better clinical response is probably a consequence of an increased dependence of the cancer cell on the prosurvival signal of the constitutive active EGFR.

Receptor mutation was also found to be responsible for most cases of acquired drug resistance against EGFR inhibitors. Especially, substitution of a single amino acid - threonine 790 - with methionine (T790M) was described in 50% of the patients who became resistant to TKI therapy after initial response. Replacement of the threonine affects the gatekeeper residue in the catalytic domain of the EGFR leading to disadvantages for the EGFR TKIs. On the one hand, sterical hindrance weakens the interactions of the inhibitor with its target. On the other hand, this mutation increases the ATP binding affinity of the EGFR leading to a disadvantage for the TKIs in the protein binding competition with ATP. Notably, although T790M alone has no strong effect on the EGFR function, combination with the characteristic activating mutations ΔE746-A750 and L858R has been shown to strongly increase the EGFR activity resulting in dramatically enhanced ligand-independent signaling.

In addition to resistance development, the occurrence of profound side effects belong to the most important limitations of EGFR-targeting therapy. Especially skin toxicity is a class-specific side effect of all EGFR inhibitors caused by their nonselective activity leading also to the inhibition of the EGF in the skin, where it is crucial for the normal development and physiology of the epidermis. Thus, it has been already recognized that new strategies to overcome these limitations and to generate better tolerable targeted drugs are urgently needed.

To address the problem of resistance, several strategies have been followed in NSCLC treatment, including the design of agents that 1) bind the EGFR tyrosine kinase irreversibly (e.g. canertinib, neratinib and EKI-785), 2) inhibit multiple members of the HER family (e.g. MP-412, a dual EGFR and HER2 tyrosine kinase inhibitor) or 3) inhibit members of multiple receptor families for targeting more than one signaling pathway (e.g. BMS-690514, a HER and VEGFR family tyrosine kinase inhibitor).

As T790M accounts for about half of all cases of acquired EGFR TKI-resistance, intensive efforts have been directed towards the development of irreversible EGFR inhibitors which are not affected by steric interference with drug binding caused by this mutation. Most of these new-generation drugs have an electrophilic functional group at the 6- or 7-position, which forms a covalent bond with a cysteine residue at the entrance of the ATP binding pocket.

Besides their ability to overcome T790M-mediated resistance, these new compounds also have other advantages, such as a long lasting drug effect or their improved selectivity for EGFR and HER2, as these are the only members of the HER family having this crucial cysteine at accessible position.

Due to these advantages, several irreversible inhibitors, including EKI-785, canertinib, neratininb, and pelitinib, have recently progressed to preclinical or clinical investigation. However, the intrinsic reactivity of the electrophilic α,β-unsaturated carbonyl moiety (which is necessary for the irreversible protein binding) is also able to act as Michael acceptor in diverse other enzymatic reactions. Consequently, for the class of irreversible EGFR inhibitors, higher rates of metabolic degradation and increased toxicity were suggested. Indeed, several clinical studies revealed that irreversible TKIs showed only limited EGFR-inhibitory activity and their application was linked with higher toxicity. Therefore, new therapeutic approaches dealing with reduction of side effects for also for this class of EGFR inhibitors are still needed.

One promising approach to reduce adverse effects of anticancer agents is the increase of tumor selectivity by the use of prodrug systems. This concept is based on non-toxic compounds which release the corresponding active drugs only after specific activation in cancerous tissues. For prodrug activation several cancer-targeting mechanisms have been reported, one of which being activation by reduction in the inherently hypoxic tissue of most solid tumours. This concept is of additional interest, since mainly tumour cells that grow under anaerobic conditions develop resistance to chemotherapeutic agents. Specific Co(III) compounds have been proposed for use as bioreducible prodrugs due to the Co(III)/Co(II) redox potential. Consequently, several Co(III) complexes have been described as hypoxia-selective anti-tumour agents, which is of interest as the majority of solid tumours contain either chronically or transiently hypoxic cells (i.e. cells lacking oxygen) to a certain extent. This is caused by the fast and uncontrolled growth of tumour tissue in contrast to the comparatively slow development of its neoplastic vascular network, as well as due to compression of the latter by the growing tumour. In addition, the concept of targeting hypoxic tissue is of great interest, as mainly tumor cells that grow under anaerobic conditions develop resistance to chemotherapeutic agents. The reasons are among others: their quiescent state (meaning that they are not dividing), their reverse transmembrane pH gradient and their inaccessibility to drugs. A particular attraction of this approach is that hypoxia-selective anti-tumor agents are expected to be selectively toxic only to tumours, as nearly all normal tissues are well oxygenated. The general mechanism of selective hypoxic activation is a one-electron bioreduction by endogenous enzymes, like the one-electron reductase NADPH-cytochrome P450 oxidoreductase (CYPOR), to an intermediate which can be easily reoxidized by molecular oxygen in normal tissue but not in hypoxic cells. This mechanism is also possible in the case of the Co³⁺/Co²⁺ redox couple. As mentioned before, octahedral Co(III) compounds are kinetically inert, meaning that their ligand configuration is expected to remain more or less intact in biological environments. If a Co(III) complex is reduced to Co(II) in normal tissue, the existing molecular oxygen immediately re-oxidizes it to the initial Co(III) complex. However, if this reduction occurs in hypoxic surroundings, the resulting labile d⁷ Co(II) species undergoes facile ligand substitution by water with release of the initially coordinated ligands.

In literature, several Co(III) complexes have been synthesized and have shown to be biologically reducible to Co(II). However, the first approaches using monodentate ligands, like aziridines, failed to be hypoxia-selective because of the insufficient stability of their corresponding Co(II) species for re-oxidation in oxygenated cells.

Since chelating ligands increase the kinetic stability of Co(II) complexes, further experiments were performed on cytotoxic agents with chelating properties. Especially nitrogen mustard complexes attracted considerable attention, as they were shown to provide both hypoxic selectivity and cytotoxic activity of the released ligands.

With these interesting results in mind, and following recent findings that hypoxia enhances RTK-mediated signaling, the present inventors developed a class of next-generation EGFR inhibitors that combine the strategies of hypoxia selectivity and EGFR inhibition, by the synthesis of Co(III) complexes with TKI chelate ligands.

Therefore, the present invention provides a compound comprising a Co³⁺ ion and a tyrosine kinase inhibitor moiety, wherein the tyrosine kinase inhibitor moiety comprises a Co³⁺-chelating moiety, and wherein the compound is bis(2,4-pentanedionato) N⁶-(2-aminoethyl)- N⁴-(3-bromophenyl)quinazoline-4,6-diamine cobalt(III) hexafluorophosphate or bis(2,4-pentanedionato) N⁶-(2-aminoethyl)-N⁴-(3-bromophenyl) quinazoline-4,6-diamine cobalt(III) chloride, according to the following Formula (A):

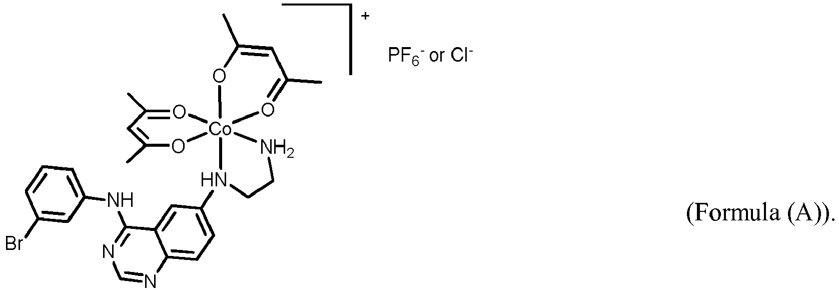

With the present invention, new derivatives of the already known EGFR inhibitors, especially erlotinib and EKI-785 are provided that comprise different bidentate chelating moieties. These new compounds have been synthesized and intensively characterized structurally (e.g. by 1D/2D NMR spectroscopy and elemental analysis) and functionally (by appropriate biological testing). The compounds were tested for their cytotoxic activity and kinase inhibition properties in cell culture as well as in xenograft experiments using SCID mice. Surprisingly, compounds according to the present invention showed increased tumor selectivity of tyrosine kinase inhibitors via coordination to Co³⁺ (with subsequent release of the inhibitor in the hypoxic tissue), leading to a reduced toxicity of such compounds compared to established TKIs, such as erlotinib.

With the data disclosed in the present invention it is possible to provide a completely new class of Co³⁺-containing antitumor drugs, namely compounds that comprise a Co³⁺ ion and a tyrosine kinase inhibitor moiety, wherein the tyrosine kinase inhibitor moiety comprises a Co³⁺-chelating moiety. This new class of compounds enabled the combination of the effectiveness of tyrosine kinase inhibitors with the advantageous properties of Co(III) compounds, especially their tumor-targeting properties due to selective activation of the complexes in a hypoxic cell environment which is frequently observed in solid tumors. This is based on the very high stability of Co(III) complexes and the easy release of the ligands of such Co(III) complexes after selective reduction in the hypoxic milieu to their labile Co(II) counterparts.

Since TKIs that are based on a 4-anilinoquinazoline structure have already been successfully applied in various antitumor treatments, such inhibitors are specifically preferred. A preferred compound according to the present invention is therefore a compound, wherein the TKI moiety is a 4-anilinoquinazoline tyrosine kinase inhibitor moiety.

Such preferred compounds according to the present invention can have a 4-anilinoquinazoline tyrosine kinase inhibitor moiety of the general formula (B), which includes a Co³⁺-chelating moiety, 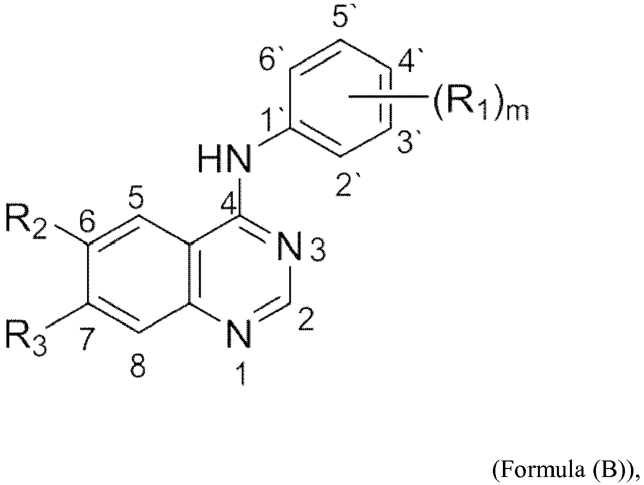 wherein m is 0, 1, 2, or 3
each R₁ is independently selected from the group consisting of amino, hydrogen, halogen, hydroxy, nitro, carboxy, formyl, cyano, trifluoromethyl, trifluoromethoxy, carbamoyl, ureido, guanidino, C₁₋₈ alkyl, C₂₋₈ alkenyl, C₂₋₈ alkynyl, C₁₋₈ alkoxy, C₃₋₈ cycloalkoxyl, C₁₋₈ alkylcarbonyl, phenyl, phenoxy, piperidino, morpholino, thiomorpholino or 2-fluorobenzyloxy, 3-fluorobenzyloxy, 4-fluorobenzyloxy;
and
R₂ and R₃ together form a Co³⁺-chelating moiety, or
R₂ and R₃ are individually selected from the group consisting of: a chelating moiety or hydrogen, alkyl, alkoxyalkyl, alkylthio, cycloalkyl, hydroxy, alkoxy, alkoxyalkyl, aralkoxy, aryl, halo, haloalkyl, carboxy, carbalkoxy CH₃CH₂O(CH₂CH₂O)_{z}CH₂CH₂ (with z = 0-5), CH₃O(CH₂CH₂O)_{z}CH₂CH₂ (with z = 0-5) and Formula (C), wherein (C) can be any of (C1) to (C6), 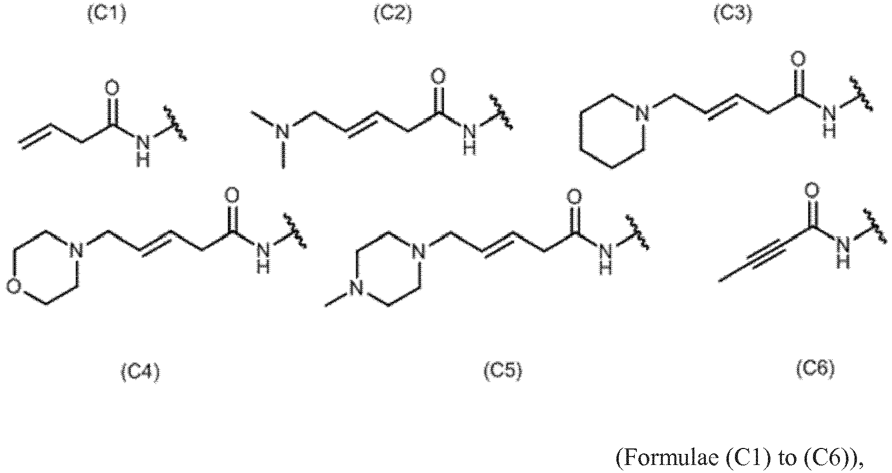 with the proviso that at least one of R₂ or R₃ is a Co³⁺-chelating moiety or R₂ and R₃ together form a Co³⁺-chelating moiety.

According to a preferred embodiment, the Co³⁺-chelating moiety is a bidentate chelating moiety. For the purpose of the present invention, the term "bidentate chelating moiety" as part of a given compound refers to two separate donor atom sites in the same compound for coordination to one metal centre.

Preferably, the Co³⁺-chelating moiety in the compound according to the present invention is covalently linked to the 4-anilinoquinazoline tyrosine kinase inhibitor moiety via C⁶ or C⁷ of the quinazoline ring in Formula (B) and that the Co³⁺-chelating moiety is selected from the following Formulae (D1) to (D6), 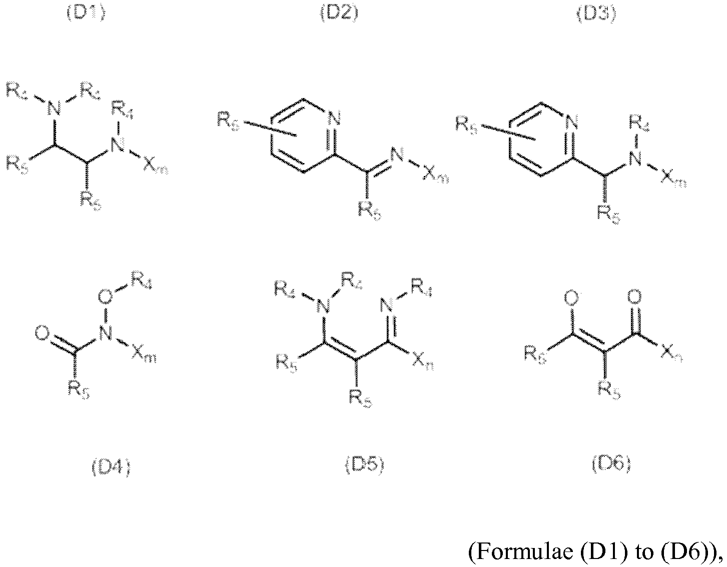 wherein m is 0 or 1 and n is 1;
R₄ is independently selected from hydrogen or C₁₋₈alkyl;
R₅ is independently selected from hydrogen, halogen, hydroxy, nitro, carboxy, formyl, cyano, trifluoromethyl, trifluoromethoxy, carbamoyl, ureido, guanidino, C₁₋₈alkyl, alkenyl, alkynyl, alkylthio, cycloalkyl, alkoxy, alkoxyalkyl, aralkoxy, aryl, haloalkyl, a phenyl group or a 5- or 6-membered heterocyclic ring containing 1 to 4 heteroatoms selected from N, O, or S; and
wherein X is C₁₋₈ alkyl, carbamoyl-C₁₋₈ alkyl, N-C₁₋₈ alkylcarbamoyl-C₁₋₈ alkyl, N-C₁₋₈ alkylcarbamoyl or CH₂CH₂O(CH₂CH₂O)_{z}CH₂CH₂ (with z = 0-5).

A specifically preferred compound according to the present invention is a compound, wherein the TKI moiety is N⁶-(2-aminoethyl)-N⁴-(3-bromophenyl)quinazoline-4,6-diamine.

According to a specifically preferred embodiment, the present invention relates to the compounds bis(2,4-pentanedionato) N⁶-(2-aminoethyl)- N⁴-(3-bromophenyl)quinazoline-4,6-diamine cobalt(III) chloride and bis(2,4-pentanedionato) N⁶-(2-aminoethyl)- N⁴-(3-bromophenyl)quinazoline-4,6-diamine cobalt(III) hexafluorophosphate.

These compounds have been proven to have significantly preferred properties and efficiencies as antitumor agents.

It is known that octahedral Co³⁺ requires six coordinative bonds so that a complex with a ligand with a bidentate chelating moiety needs four additional coordinative bonds. This can be effected by four monodentate ligands, two (further) bidentate ligands, a monodentate ligand and a tridentate ligand, or a tetradentate ligand.

The present invention also relates to compounds or complexes comprising a compound of the invention and one or more ligands capable of forming a coordinative bond with a Co³⁺ ion. Preferably the complexes according to the present invention comprise one, two, three or four mono-, bi- or tetradentate ligand(s) in order to attain a total of six coordinative bonds. In one embodiment, the complexes of the invention may comprise one or two additional 4-anilinoquinazoline tyrosine kinase inhibitors (bidentate ligands).

Preferably, the compound according to the present invention comprises a further component, which is a ligand independently selected from the group consisting of compounds of Formulae (E1) to (E5): 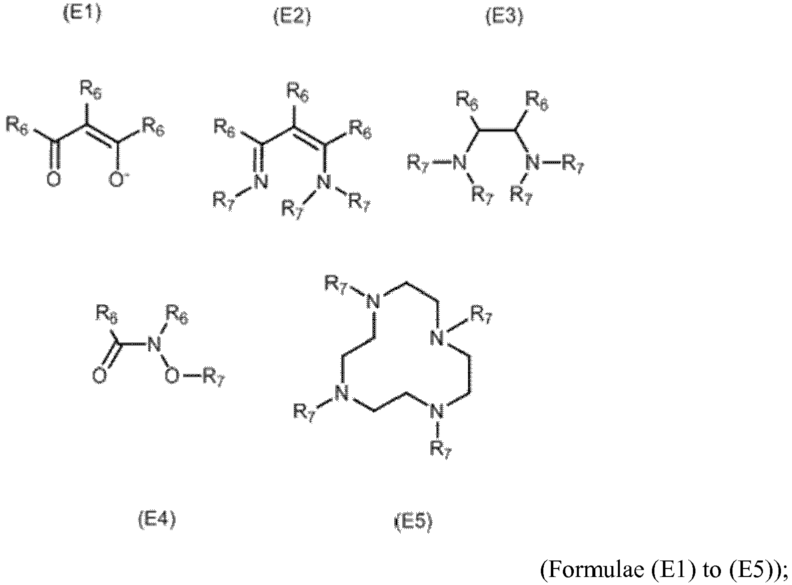 halido, nitrite, and ammine;
wherein R₆ is independently selected from the group consisting of hydrogen, halogen, hydroxy, nitro, carboxy, formyl, cyano, trifluoromethyl, trifluoromethoxy, carbamoyl, ureido, guanidino, alkyl, alkenyl, alkynyl, alkylthio, cycloalkyl, alkoxy, alkoxyalkyl, aralkoxy, aryl, and haloalkyl;
and R₇ is independently selected from the group consisting of hydrogen and C₁₋₈ alkyl.

Since the compounds according to the present invention can have one or more counter ions (cations or anions), certain preferred embodiments of the compounds according to the present invention have such counter ions, preferably 0, 1,2, or 3 counter ions. Preferred compounds of the present invention can therefore comprise a counter ion Yₙ (with n is 0-3), which is independently selected from chloride, bromide, carbonate, RCOO⁻ (with R = C₁₋₆alkyl, C₁₆alkenyl), sulfate, phosphate, methanesulphonate, trifluorosulphonate, benzenesulfanate, p-toluene sulphonate, 1-napthalene sulfonate, acetate, trifluoroacetate, malate, tartrate, citrate, lactate, oxalate, salicylate, hydrogen, sodium, potassium, ammonium, phosphonium, pyrrolidinium, pyridinium, and imidazolium.

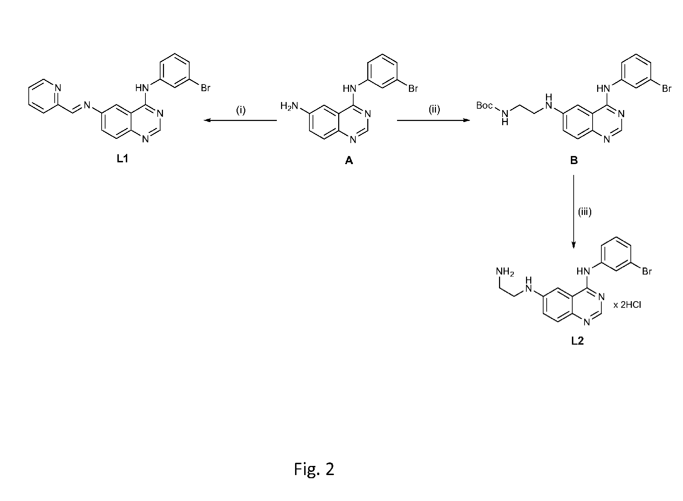

In a further aspect, the invention relates to the compound L2 as depicted in Figure 2, which is a novel 4-anilinoquinazoline tyrosine kinase inhibitor moiety with *in vitro* efficacy comparable to erlotinib (Table 1). The invention further relates to L2 for use as a medicament.

In yet another aspect, the invention relates to methods of synthesis of the compounds and complexes of the invention. The skilled person is capable of synthesizing such compounds and complexes from suitable precursors.

In one particular embodiment the invention concerns a method for synthesizing compound L2 comprising the steps of: (i) direct reductive amination of N-Boc-2-aminoacetaldehyde with compound A (Figure 2); and (ii) deprotection with hydrochloric acid.

In another specific embodiment the invention relates to a method for synthesizing the complex of Formula (A) comprising the steps of: (i) treating hexanitrocobaltate (III) with an aqueous solution of acetylacetone to form compound D; and (ii) reacting D and L2 in the presence of activated charcoal using a mixture of water and methanol as solvents.

The compounds according to the present invention are preferably used in a therapeutic treatment. According to a preferred aspect of the present invention, the present compounds are used for the treatment of a hyperproliferative disorder in a mammal which comprises administering to said mammal a pharmaceutically effective amount of the compound of the present invention. The compounds according to the present invention are specifically suitable for use in the treatment of carcinomas, myeloid disorders or adenomas, especially for use in the treatment of cancer, preferably non-small cell lung cancer (NSCLC), head and neck cancer, colorectal cancer, prostate cancer, bladder cancer, esophageal cancer, pancreatic cancer, gastroesophageal cancer, ovarian cancer, cervical cancer, astrocytoma grade 1-4, renal cell cancer, and prostate cancer.

In one embodiment the compounds of the invention are for use in treating solid tumours, particularly for larger solid tumours, such as tumours of size T-2, T-3, or T-4, according to the TNM tumour grading system.

The compounds of the invention can be used for treating patients in whom first-line EGFR inhibitor therapy has failed (e.g. due to development of resistance). The compounds of the invention are particularly useful for treating EGFR-driven cancer types resulting from EGFR overexpression (wild-type or carrying an erlotinib-sensitizing mutation) or for treatment of cancerous cells carrying an erlotinib resistance-mediating secondary EGFR mutation.

Thus, compounds of the invention can be used to treat patients in whom cells carry EGFR mutations, including Exon 19 deletion, L858R, T790M, G719X, S768I, and L861Q mutations. In a preferred embodiment the compounds of the invention can be used to target hyperproliferative cells carrying a T790M mutation. In another preferred embodiment the compounds of the invention can be used to target hyperproliferative cells carrying a T790M mutation, an L858R mutation and a ΔE746-A750 mutation.

In one embodiment, the invention concerns a method for treating a cancer patient comprising:
(i) Identification of EGFR gene mutations in an overproliferating cell type or tumour extracted from the patient; and
(ii) Administering a compound according to the invention to the patient.

The compound according to the present invention produces a receptor tyrosine kinase inhibitory effect in humans or warm-blooded animals in need of such treatment which comprises administering to said animal an effective amount. As used herein, the term "effective amount" refers to the amount of a compound provided herein which is sufficient to reduce or ameliorate the severity, duration of a disorder, cause regression of a disorder, prevent the recurrence, development, or onset of one or more symptoms associated with a disorder, or enhance or improve the therapeutic effect(s) of another therapy.

In a specific embodiment, with respect to the treatment of cancer, an effective amount of a compound according to the present invention refers to the amount of a therapy that inhibits or reduces the proliferation of cancerous cells, inhibits or reduces the spread of tumour cells (metastasis), inhibits or reduces the onset, development or progression of one or more symptoms associated with cancer, or reduces the size of a tumour. In certain embodiments, a therapeutically effective of a therapy reduces the proliferation of cancerous cells or the size of a tumour by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%), at least 90%, at least 95%, or at least 99%, relative to a control or placebo such as phosphate buffered saline ("PBS").

The terms "therapies" and "therapy" can refer to any protocol(s), method(s), and/or agent(s) that can be used in the prevention, treatment, management, or amelioration of a disorder or one or more symptoms thereof. In certain embodiments, the terms "therapy" and "therapies" refer to a combination of administering the compound according to the present invention with another antitumour therapy, for example chemotherapy, radiation therapy, hormonal therapy, biological therapy, and/or other therapies useful in the prevention, management, treatment or amelioration of a neoplastic disorder (tumour, cancer).

As used herein, the terms "treat", "treatment" and "treating" refer to the reduction or amelioration of the progression, severity and/or duration of a disorder, or the amelioration of one or more symptoms thereof resulting from the administration of one or more therapies.

As used herein, the term "modulation" or "modulating" refers to the alteration of the catalytic activity of a tyrosine kinase. In particular, modulating can refer to the activation or to the inhibition of the tyrosine kinase. The tyrosine kinase can be any tyrosine kinase known to those of skill in the art. In certain embodiments, the tyrosine kinase is a receptor tyrosine kinase or an intracellular tyrosine kinase.

The compound according to the present invention is also used for treating in humans or a warm-blooded animal a disease or medical condition mediated alone or in part by the enzyme receptor tyrosine kinase.

Subject to the treatment of the present invention are preferably human patients. These terms "subject" and "individual" can be used interchangeably with "patient". The terms "patient" and "patients" refer to an animal, in certain embodiments a mammal including a non-primate (e.g., a cow, pig, horse, cat, dog, rat, and mouse) and a primate (e.g., a monkey such as a cynomolgous monkey, a chimpanzee and a human), and more particularly a human. In another embodiment, the subject is a farm animal (e.g., a horse, a cow, a pig, etc.) or a pet (e.g., a dog or a cat).

The present invention also relates to a pharmaceutical formulation comprising at least one compound of the present invention or a pharmaceutically acceptable salt thereof, together with one or more pharmaceutically acceptable carriers, diluents or excipients.

Pharmaceutical "carriers", "diluents" and "excipients" are substances to be admixed in a pharmaceutical composition as auxiliary substances to the compound according to the present invention or other compounds with pharmaceutical effect (e.g. that have also antitumor effects). These auxiliary substances do not have an effect on their own (they are no "active substance" per se) but may assist to optimize the effectivity of the compound according to the present invention. The substances used as "carriers", "diluents" and excipients" can often be used interchangeable (i.e. that substances that are used as "diluents" may also serve as "excipients" and/or "carriers".

In general, "carriers" are substances that improve the delivery and the effectiveness of drugs; "diluents" are substances that dilute the active substance in a pharmaceutical preparation; "excipients" are substances that are admixed to the pharmaceutical formulation for defining its releasing properties.

All such "carriers", "diluents" and "excipients" (i.e. auxiliary substances, sometimes also the term "excipient" is used as the general term including all such substances) are, as already stated, inactive substances formulated alongside the active ingredient ("API") of a medication, for the purpose of bulking-up formulations that contain potent active ingredients (thus often also referred to as "bulking agents", "fillers", or "diluents"). Bulking up allows convenient and accurate dispensation of a drug substance when producing a dosage form. They also can serve various therapeutic-enhancing purposes, such as facilitating drug absorption or solubility, or other pharmacokinetic considerations. Such substances can also be useful in the manufacturing process, to aid in the handling of the active substance concerned such as by facilitating powder flowability or non-stick properties, in addition to aiding in vitro stability such as prevention of denaturation over the expected shelf life. The selection of appropriate excipients, carriers and diluents also depends upon the route of administration and the dosage form, as well as the active ingredient and other factors. Such substances e.g. serve as antiadherents, binders coatings, disintegrants, fillers, flavours, colours, lubricants, glidants, sorbents, preservatives, sweeteners, etc.

Drug "carriers" are substances that serve as mechanisms to improve the delivery and the effectiveness of drugs. Drug carriers are used in sundry drug delivery systems such as: controlled-release technology to prolong in vivo drug actions; decrease drug metabolism, and reduce drug toxicity.

Carriers are also used in designs to increase the effectiveness of drug delivery to the target sites of pharmacological actions.

Drug "diluents" are usually substances that simply dilute or reconstitute a pharmaceutical composition (e.g. after storage in dry form).

The pharmaceutical composition with the compound according to the present invention usually comprises the compound according to the present invention in an amount of 0.01 (%w/w) to 80 (%w/w) in a solid composition or 0.001 (%w/v) to 80 (%w/v) in a liquid composition. Preferably the compound according to the present invention is present in an amount of 0.1 (%w/w) to 50 (%w/w) in a solid composition or 0.01 (%w/v) to 10 (%w/v) in a liquid composition, especially of 1 (%w/w) to 20 (%w/w) in a solid composition or 0.1 (%w/v) to 5 (%w/v) in a liquid composition.

Preferably, the compounds according to the present invention are provided as a pharmaceutical composition, especially as a pharmaceutical single unit dosage form. Pharmaceutical compositions and single unit dosage forms provided herein comprise a prophylactically or therapeutically effective amount of one or more prophylactic or therapeutic agents (e.g., a compound provided herein, or other prophylactic or therapeutic agent), and one or more pharmaceutically acceptable carriers or excipients or diluents. In a specific embodiment and in this context, the term "pharmaceutically acceptable" means approved by a regulatory agency of the Federal or a state government or listed in the European or U.S. Pharmacopeia or other generally recognized pharmacopeia for use in animals, and more particularly in humans.

The term "carrier" refers to a diluent, adjuvant (e.g., Freund's adjuvant (complete and incomplete)), or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a particular carrier when the pharmaceutical composition is administered intravenously. Saline solutions and aqueous dextrose and glycerol solutions can also be employed as liquid carriers, particularly for injectable solutions.

On one embodiment, pharmaceutical compositions, and dosage forms comprise one or more excipients. Suitable excipients are well-known to those skilled in the art of pharmacy, and non-limiting examples of suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Whether a particular excipient is suitable for incorporation into a pharmaceutical composition or dosage form depends on a variety of factors well known in the art including, but not limited to, the way in which the dosage form will be administered to a patient and the specific active ingredients in the dosage form. The composition or single unit dosage form, if desired, can also contain minor amounts of wetting or emulsifying agents, or pH buffering agents.

Lactose-free compositions provided herein can comprise excipients that are well known in the art and are listed, for example, in the U.S. Pharmacopeia (USP) SP (XXI)/NF (XVI). In one embodiment, lactose-free compositions comprise an active ingredient, a binder/filler, and a lubricant in pharmaceutically compatible and pharmaceutically acceptable amounts. Exemplary lactose-free dosage forms comprise an active ingredient, microcrystalline cellulose, pre-gelatinized starch, and magnesium stearate.

Provided herein are anhydrous pharmaceutical compositions and dosage forms comprising active ingredients, since water can facilitate the degradation of some compounds. For example, the addition of water (e.g. 5%) is widely accepted in the pharmaceutical arts as a means of simulating long-term storage in order to determine characteristics such as shelf-life or the stability of formulations over time. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms provided herein can be prepared using anhydrous or low moisture containing ingredients and low moisture or low humidity conditions. Pharmaceutical compositions and dosage forms that comprise lactose and at least one active ingredient that comprises a primary or secondary amine are, in certain embodiments, anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions are in certain embodiments packaged using materials known to prevent exposure to water such that they can be included in suitable formulary kits. Examples of suitable packaging include, but are not limited to, hermetically sealed foils, plastics, unit dose containers (e.g., vials), blister packs, and strip packs.

Provided herein are pharmaceutical compositions and dosage forms that comprise one or more compounds that reduce the rate by which an active ingredient will decompose. Such compounds, which are referred to herein as "stabilizers," include, but are not limited to, antioxidants such as ascorbic acid, pH buffers, or salt buffers.

The pharmaceutical compositions and single unit dosage forms can take the form of solutions, suspensions, emulsion, tablets, pills, capsules, powders, sustained-release formulations and the like. Oral formulation can include standard carriers such as pharmaceutical grades of mannitol, lactose, starch, magnesium stearate, sodium saccharine, cellulose, magnesium carbonate, etc. Such compositions and dosage forms will contain a prophylactically or therapeutically effective amount of a prophylactic or therapeutic agent in certain embodiments in purified form, together with a suitable amount of carrier so as to provide the form for proper administration to the patient. The formulation should suit the mode of administration. In certain embodiments, the pharmaceutical compositions or single unit dosage forms are sterile and in suitable form for administration to a subject, in certain embodiments an animal subject, such as a mammalian subject, particularly a human subject.

A pharmaceutical composition provided herein is formulated to be compatible with its intended route of administration. Examples of routes of administration include, but are not limited to parenteral, e.g., intravenous, intradermal, subcutaneous, intramuscular, subcutaneous, oral, buccal, sublingual, inhalation, intranasal, transdermal, topical, trans-mucosal, intra-tumoural, intra-synovial and rectal administration.

In a specific embodiment, the composition is formulated in accordance with routine procedures as a pharmaceutical composition adapted for intravenous, subcutaneous, intramuscular, oral, intranasal or topical administration to human beings.

In an embodiment, a pharmaceutical composition is formulated in accordance with routine procedures for subcutaneous administration to human beings. In one embodiment, compositions for intravenous administration are solutions in sterile isotonic aqueous buffer. Where necessary, the composition may also include a solubilizing agent and a local anaesthetic such as lignocaine to ease pain at the site of the injection.

Examples of dosage forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (e.g., nasal sprays or inhalers); gels; liquid dosage forms suitable for oral or mucosal administration to a patient, including suspensions (e.g., aqueous or non-aqueous liquid suspensions, oil-in-water emulsions, or a water-in-oil liquid emulsions), solutions, and elixirs; liquid dosage forms suitable for parenteral administration to a patient; and sterile solids (e.g., crystalline or amorphous solids) that can be reconstituted to provide liquid dosage forms suitable for parenteral administration to a patient.

The composition, shape, and type of dosage forms provided herein will typically vary depending on their use. For example, a dosage form used in the acute treatment of inflammation or a related disorder may contain larger amounts of one or more of the active ingredients it comprises than a dosage form used in the chronic treatment of the same disease. Also, the therapeutically effective dosage form may vary among different types of cancer. Similarly, a parenteral dosage form may contain smaller amounts of one or more of the active ingredients it comprises than an oral dosage form used to treat the same disease or disorder. These and other ways in which specific dosage forms provided herein will vary from one another will be readily apparent to those skilled in the art.

The ingredients of compositions provided herein are supplied either separately or mixed together in unit dosage form, for example, as a dry lyophilized powder or water-free concentrate in a hermetically sealed container such as an ampoule or sachette ndicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

Typical dosage forms comprise a compound provided herein, or a pharmaceutically acceptable salt, solvate or hydrate thereof lie within the range of from about 0.1 mg to about 1000 mg per day. Particular dosage forms have about 0.1, 0.2, 0.3, 0.4, 0.5, 1.0, 2.0, 2.5, 5.0, 10.0, 15.0, 20.0, 25.0, 50.0, 100, 200, 250, 500, or 1000 mg of the compound.

Pharmaceutical compositions provided herein that are suitable for oral administration can be presented as discrete dosage forms, such as, but are not limited to, tablets (e.g., chewable tablets), caplets, capsules, and liquids (e.g., flavoured syrups). Such dosage forms contain predetermined amounts of active ingredients, and may be prepared by methods of pharmacy well known to those skilled in the art. In certain embodiments, the oral dosage forms are solid and prepared under anhydrous conditions with anhydrous ingredients, as described in detail in the sections above. However, the scope extends beyond anhydrous, solid oral dosage forms. As such, further forms are described herein.

Typical oral dosage forms provided herein are prepared by combining the active ingredient(s) in an intimate admixture with at least one excipient according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavouring agents, preservatives, and colouring agents. Examples of excipients suitable for use in solid oral dosage forms (e.g., powders, tablets, capsules, and caplets) include, but are not limited to, starches, sugars, micro-crystalline cellulose, diluents, granulating agents, lubricants, binders, and disintegrating agents.

Because of their ease of administration, tablets and capsules represent the most advantageous oral dosage unit forms, in which case solid excipients are employed. If desired, tablets can be coated by standard aqueous or non-aqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine, a mixture of the powdered compound moistened with an inert liquid diluent.

Examples of excipients that can be used in oral dosage forms include, but are not limited to, binders, fillers, disintegrants, and lubricants. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (e.g., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (e.g., Nos. 2208, 2906, 2910), microcrystalline cellulose, and mixtures thereof.

Examples of fillers suitable for use in the pharmaceutical compositions and dosage forms disclosed herein include, but are not limited to, talc, calcium carbonate (e.g., granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions provided herein is typically present in from about 50 to about 99 weight percent of the pharmaceutical composition or dosage form.

Suitable forms of microcrystalline cellulose include, but are not limited to, the materials sold as AVICEL-PH-101, AVICEL-PH-103 AVICEL RC-581, AVICEL-PH-105 (available from FMC Corporation, American Viscose Division, Avicel Sales, Marcus Hook, PA), and mixtures thereof. A specific binder is a mixture of microcrystalline cellulose and sodium carboxymethyl cellulose sold as AVICEL RC-581. Suitable anhydrous or low moisture excipients or additives include AVICEL-PH-103™ and Starch 1500 LM.

Disintegrants are used in the compositions to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms provided herein. The amount of disintegrant used varies based upon the type of formulation, and is readily discernible to those of ordinary skill in the art. Typical pharmaceutical compositions comprise from about 0.5 to about 15 weight percent of disintegrant, specifically from about 1 to about 5 weight percent of disintegrant.

Disintegrants that can be used in pharmaceutical compositions and dosage forms include, but are not limited to, agar-agar, alginic acid, calcium carbonate, microcrystalline cellulose, croscarmellose sodium, crospovidone, polacrilin potassium, sodium starch glycolate, potato or tapioca starch, pre-gelatinized starch, other starches, clays, other algins, other celluloses, gums, and mixtures thereof.

Lubricants that can be used in pharmaceutical compositions and dosage forms include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL 200, manufactured by W.R. Grace Co. of Baltimore, MD), a coagulated aerosol of synthetic silica (marketed by Degussa Co. of Piano, TX), CAB-O-SIL (a pyrogenic silicon dioxide product sold by Cabot Co. of Boston, MA), and mixtures thereof. If used at all, lubricants are typically used in an amount of less than about 1 weight percent of the pharmaceutical compositions or dosage forms into which they are incorporated.

Active ingredients such as the compounds provided herein can be administered by controlled release means or by delivery devices that are well known to those of ordinary skill in the art. Such dosage forms can be used to provide slow or controlled-release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled-release formulations known to those of ordinary skill in the art, including those described herein, can be readily selected for use with the active ingredients. Thus provided are single unit dosage forms suitable for oral administration such as, but not limited to, tablets, capsules, gel caps, and caplets that are adapted for controlled-release.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood levels of the drug, and can thus affect the occurrence of side (e.g., adverse) effects.

Most controlled-release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds.

Parenteral dosage forms can be administered to patients by various routes including, but not limited to, subcutaneous, intravenous (including bolus injection), intramuscular, and intraarterial. Because their administration typically bypasses patients' natural defences against contaminants, parenteral dosage forms are in certain embodiments sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms are well known to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, com oil, cottonseed oil, peanut oil, sesame oil, ethyl oleate, isopropyl myristate, and benzyl benzoate.

Compounds that increase the solubility of one or more of the active ingredients disclosed herein can also be incorporated into the parenteral dosage forms.

Transdermal, topical, and mucosal dosage forms provided herein include, but are not limited to, ophthalmic solutions, sprays, aerosols, creams, lotions, ointments, gels, solutions, emulsions, suspensions, or other forms known to one of skill in the art. Dosage forms suitable for treating mucosal tissues within the oral cavity can be formulated as mouthwashes or as oral gels. Further, transdermal dosage forms include "reservoir type" or "matrix type" patches, which can be applied to the skin and worn for a specific period of time to permit the penetration of a desired amount of active ingredients.

Suitable excipients (e.g., carriers and diluents) and other materials that can be used to provide transdermal, topical, and mucosal dosage forms provided herein are well known to those skilled in the pharmaceutical arts, and depend on the particular tissue to which a given pharmaceutical composition or dosage form will be applied. With that fact in mind, typical excipients include, but are not limited to, water, acetone, ethanol, ethylene glycol, propylene glycol, butane- 1,3-diol, isopropyl myristate, isopropyl palmitate, mineral oil, and mixtures thereof to form lotions, tinctures, creams, emulsions, gels or ointments, which are non-toxic and pharmaceutically acceptable. Moisturizers or humectants can also be added to pharmaceutical compositions and dosage forms if desired, Examples of such additional ingredients are well known in the art. Depending on the specific tissue to be treated, additional components may be used prior to, in conjunction with, or subsequent to treatment with active ingredients provided herein. For example, penetration enhancers can be used to assist in delivering the active ingredients to the tissue. Suitable penetration enhancers include, but are not limited to: acetone; various alcohols such as ethanol, oleyl, and tetrahydrofuryl; alkyl sulfoxides such as dimethyl sulfoxide; dimethyl acetamide; dimethyl formamide; polyethylene glycol; pyrrolidones such as polyvinylpyrrolidone; Kollidon grades (Povidone, Polyvidone); urea; and various water-soluble or insoluble sugar esters such as Tween 80 (polysorbate 80) and Span 60 (sorbitanmonostearate).

The pH of a pharmaceutical composition or dosage form, or of the tissue to which the pharmaceutical composition or dosage form is applied, may also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different salts, hydrates or solvates of the active ingredients can be used to further adjust the properties of the resulting composition.

The amount of the compound or composition which will be effective in the prevention, treatment, management, or amelioration of a disorder or one or more symptoms thereof will vary with the nature and severity of the disease or condition, and the route by which the active ingredient is administered. The frequency and dosage will also vary according to factors specific for each patient depending on the specific therapy (e.g., therapeutic or prophylactic agents) administered, the severity of the disorder, disease, or condition, the route of administration, as well as age, body, weight, response, and the past medical history of the patient. Effective doses may be extrapolated from dose-response curves derived from in vitro or animal model test systems.

Exemplary doses of a compound include milligram or microgram amounts of the active peptide per kilogram of subject or sample weight (e.g., about 1 mg per kg to about 500 mg per kg, about 100 µg per kg to about 5 mg per kg, or about 1 µg per kg to about 50 µg per kg). In one embodiment, the recommended daily dose range of a compound provided herein for the conditions described herein lie within the range of from about 0.01 mg to about 1000 mg per day, given as a single once-a-day dose in certain embodiments as divided doses throughout a day. It may be necessary to use dosages of the active ingredient outside the ranges disclosed herein in some cases, as will be apparent to those of ordinary skill in the art. Furthermore, it is noted that the clinician or treating physician will know how and when to interrupt, adjust, or terminate therapy in conjunction with individual patient response.

Different therapeutically effective amounts may be applicable for different diseases and conditions, as will be readily known by those of ordinary skill in the art. Similarly, amounts sufficient to prevent, manage, treat or ameliorate such disorders, but insufficient to cause, or sufficient to reduce, adverse effects associated with the compounds provided herein are also encompassed by the above described dosage amounts and dose frequency schedules. Furthermore, when a patient is administered multiple dosages of a compound provided herein, not all of the dosages need be the same. For example, the dosage administered to the patient may be increased to improve the prophylactic or therapeutic effect of the compound or it may be decreased to reduce one or more side effects that a particular patient is experiencing.

In certain embodiments, administration of the same compound provided herein may be repeated and the administrations may be separated by at least 1 day, 2 days, 3 days, 5 days, 10 days, 15 days, 30 days, 45 days, 2 months, 75 days, 3 months, or 6 months. In other embodiments, administration of the same prophylactic or therapeutic agent may be repeated and the administration may be separated by at least 1 day, 2 days, 3 days, 5 days, 10 days, 15 days, 30 days, 45 days, 2 months, 75 days, 3 months, or 6 months.

Optionally, the compounds of the invention may be administered in combination with one or more other cytotoxic drugs, as are known in the art. The compounds of the invention and the one or more other cytotoxic drugs may be provided in the form of a kit, for separate, sequential or separate administration. In one embodiment, a compound of the invention is provided together with one or more other cytotoxic drugs in a single dosage pharmaceutical form.

"Pharmaceutically acceptable salt" refers to any salt of a compound provided herein which retains its biological properties and which is not toxic or otherwise undesirable for pharmaceutical use. Such salts may be derived from a variety of organic and inorganic counterions well known in the art and include. Such salts include: acid addition salts formed with organic or inorganic acids such as hydrochloric, hydrobromic, sulfuric, nitric, phosphoric, sulfamic, acetic, trifluoroacetic, trichloroacetic, propionic, hexanoic, cyclopentylpropionic, glycolic, glutaric, pyruvic, lactic, malonic, succinic, sorbic, ascorbic, malic, maleic, fumaric, tartaric, citric, benzoic, 3-(4-hydroxybenzoyl)benzoic, picric, cinnamic, mandelic, phthalic, lauric, methanesulfonic, ethanesulfonic, 1 ,2-ethane-disulfonic, 2-hydroxyethanesulfonic, benzenesulfonic, 4-chlorobenzenesulfonic, 2-naphthalenesulfonic, 4-toluenesulfonic, camphoric, camphorsulfonic, 4-methylbicyclo[2.2.2]-oct-2-ene-1-carboxylic, glucoheptonic, 3-phenylpropionic, trimethylacetic, tert-butylacetic, lauryl sulfuric, gluconic, benzoic, glutamic, hydroxynaphthoic, salicylic, stearic, cyclohexylsulfamic, quinic, muconic acid and the like acids.

Such salts further include, by way of example only, sodium, potassium, calcium, magnesium, ammonium, tetraalkylammonium and the like, and when the compound contains a basic functionality, salts of non-toxic organic or inorganic acids, such as hydrochloride, hydrobromide, tartrate, mesylate, besylate, acetate, maleate, oxalate and the like. The term "physiologically acceptable cation" refers to a non-toxic, physiologically acceptable cationic counterion of an acidic functional group. Such cations are exemplified by sodium, potassium, calcium, magnesium, ammonium and tetraalkylammonium cations and the like.

The compounds according to the present invention may also be provided as solvates. "Solvate" refers to a compound provided herein or a salt thereof that further includes a stoichiometric or non-stoichiometric amount of solvent bound by non-covalent intermolecular forces. Where the solvent is water, the solvate is a hydrate.

The present invention is further disclosed by the following examples and the figures, yet without being restricted thereto.
Fig. 1 shows a schematic overview of the TKI Co(III)-prodrug concept according to the present invention.
Fig. 2 shows the synthesis pathway from core structure A to the two compounds **L1** and L2. Reagent conditions: (i) pyridine-2-carboxaldehyde, MeOH, reflux, 94%; (ii) *N*-Boc-2-aminoacetaldehyde, abs. MeOH, HOAc, molecular sieves (3-4 Å), then NaBH₃CN, 85%; (iii) conc. HCl, EtOH, reflux, 82%.
Fig. 3 shows the inhibitory effects of the novel drugs on EGFR signaling. HCC827 cells were treated with increasing concentrations of L2, and erlotinib for 4 h and activation of EGFR downstream pathways (pERK, pEGFR) was analyzed by Western blotting.
Fig. 4 shows synthesis of complexes according to the present invention.
Fig. 5 shows anticancer activity of the novel Co(III) complex tested in comparison to the metal-free ligand under normoxic and hypoxic conditions.
Figs. 6 and 7 show hypoxia-specific activation of the complexes according to the present invention in cell culture experiments.
Fig. 8 shows activity of the complexes according to the present invention tested against EGFR-driven xenografts in SCID mice.

### EXAMPLES:

### Materials and Methods

All solvents and reagents were obtained from commercial suppliers and, unless specified differently, used without further purification. HOAc was freshly distilled. Anhydrous MeOH and THF were bought from Sigma-Aldrich over molecular sieves. Erlotinib was purchased from LC Laboratories^{®}. Compound A was synthesized according to literature methods (Tsou et al., J Med Chem 2001, 44, 2719-34). Preparative RP-HPLC was performed on an Agilent 1200 Series system controlled by Chemstation software. The experimental conditions were as follows: stationary phase: silica-based C18 gel; column: XBridge BEH C18 OBD Prep Column, 130Å, 5 µm, 19 mm x 250 mm); flow rate: 17.06 ml/min, injection volume: 5-7 ml; column temperature: 25°C. Elemental analyses were performed by the Microanalytical Laboratory of the University of Vienna. ESI-MS spectrometry was carried out with a Bruker Esquire₃₀₀₀ ion trap spectrometer (BrukerDaltonic, Bremen, Germany). Expected and experimental isotope distributions were compared. ¹H and ¹³C NMR spectra were recorded in *d₆*-DMSO, *d₇*-DMF, D₂O, MeOD or CDCl₃, referring to the respective solubility of the synthesized compounds, with a Bruker FT-NMR Avance III 500 MHz spectrometer at 500.10 (¹H) and 125.75 (¹³C) MHz at 298 K. Chemical shifts (ppm) were referenced internal to the solvent residual peaks. For the description of the spin multiplicities the following abbreviations were used: s = singlet, d = duplet, t = triplet, q = quartet, m = multiplet.

### N⁴-(3-bromophenyl)-N⁶-(pyridin-2-ylmethylene) quinazoline-4,6-diamine (L₁).

To a solution of A (469 mg, 1.49 mmol) in a minimum of hot MeOH was added drop-wise pyridin-2-carbaldehyde (710 µl, 7.43 mmol). The reaction mixture was stirred for 3 h under reflux and stored over night at room temperature. A yellow precipitate was formed which was filtered, washed with MeOH and dried in vacuo. Yield: 563 mg (94%). Anal.Calcd for C₂₀H₁₄BrN₅ · 0.5H₂O (Mᵣ = 413.27 g/mol): C, 58.13; H, 3.66; N, 16.95; O, 1.94. Found: C, 58.17; H, 3.42; N, 16.59; O, 1.98. ¹H NMR (500.1 MHz, DMSO-*d₆*): δ 7.32 (d, *J=* 7.9 Hz, 1H), 7.39 (dd, *J=* 8.0 Hz, *J=* 8.0 Hz, 1H), 7.60 (ddd, *J=* 1.2 Hz, *J=* 4.8 Hz, *J=* 7.5 Hz, 1H), 7.89 (d, *J=* 8.8 Hz, 1H), 7.95-7.97 (m, 2H), 8.02-8.05 (m, 1H), 8.25-8.28 (m, 2H), 8.55 (d, *J=* 2.0 Hz, 1H), 8.68 (s, 1H), 8.79 (d, *J*= 4.8 Hz, 1H), 8.86 (s, 1H), 9.93 (s, 1H) ppm. Data according to literature.

### tert-Butyl {2-[(4-((3-bromophenyl)amino)quinazolin-6-yl)amino]ethyl}carbamate (B).

To a solution of A (500 mg, 1.6 mmol) and HOAc (90.8 µl, 1.6 mmol) in anhydrous MeOH (16 ml) containing a 3-4 Ǻ molecular sieve was added *N*-boc-2-aminoacetaldehyde (303 mg, 1.9 mmol) and the resulting mixture was stirred under Ar at room temperature for 1 h. Sodium cyanoborohydride (120 mg, 1.9 mmol) was added and the resulting suspension was stirred at room temperature overnight. The solvent was removed under reduced pressure and the residue was dissolved in EtOAc (200 ml), washed with 1 M HCl (150 ml), sat. NaHCO₃ (150 ml), and brine (150 ml). The organic layer was dried over MgSO₄, filtered, concentrated under reduced pressure and dried in vacuo to give a bright yellow solid. Yield: 615 mg (85%). ¹H NMR (500.1 MHz, DMSO-*d₆*): δ 1.40 (s, 9H), 3.26 (s, 4H), 6.22 (s, 1H), 6.97 (s, 1H), 7.23 (s, 1H), 7.27-7.29 (m, 2H), 7.36 (dd, *J=* 8.0 Hz, *J=* 8.0 Hz, 1H), 7.57 (d, *J=* 9.0 Hz, 1H), 7.91 (d, *J =* 8.0 Hz, 1H), 8.17 (s, 1H), 8.41 (s, 1H), 9.36 (s, 1H) ppm.

### N⁶-(2-aminoethyl)-N⁴-(3-bromophenyl)quinazoline-4,6-diamine dihydrochloride (L₂).

To a solution of **B** (614 mg, 1.3 mmol) in EtOH (13.5 ml) was added conc. HCl at elevated temperature and the reaction mixture was stirred under reflux for 4 h. The solution was cooled to room temperature and stored for 10 min at 4°C. The deep yellow precipitate was filtered, washed with EtOH and dried in vacuo. Yield: 462 mg (82%). Anal.Calcd for C₁₆H₁₆BrN₅ · 2 HCl (Mᵣ = 431.16 g/mol): C, 44.57; H, 4.21; N, 16.24. Found: C, 44.54; H, 4.35; N, 16.25. ¹H NMR (500.1 MHz, DMSO-*d₆*): δ 3.05-3.08 (m, 2H, H21), 3.63 (t, ³*J*= 5.8 Hz, 2H, H20), 7.18 (s, 1H, H18), 7.45 (dd, ³*J*= 8.0 Hz, ³*J*= 8.0 Hz, 1H, H16), 7.50-7.53 (m, 2H, H2, H15), 7.78 (d, ³*J*= 9.0 Hz, 1H, H3), 7.88 (d, ³*J*= 8.0 Hz, 1H, H11), 8.02 (s, 1H, H6), 8.13 (s, 1H, H 13), 8.27 (s, 3H, H22), 8.77 (s, 1H, H8), 11.54 (s, 1H, H17) ppm. ¹³C NMR (125.75 MHz, DMSO-*d₆*): δ 37.6 (C21), 40.6 (C20), 99.1 (C6), 115.9 (C5), 121.1 (C3), 121.5 (C14), 124.3 (C11), 127.0 (C2), 127.8 (C13), 129.2 (C15), 130.9 (C16), 131.2 (C4), 139.2 (C12), 146.6 (C8), 149.2 (C1), 158.8 (C10) ppm.

### Sodium hexanitrocobaltate(III) (C).

Sodium nitrite (60 g, 0.87 mol) was dissolved in H₂O (60 ml) at 70°C and cobalt(II) nitrate hexahydrate (20 g, 69 mmol) was added in portions. Afterwards HOAc (50%, 20 ml) was added drop-wise and air was bubbled through the reaction mixture for 30 min. At 0°C EtOH (100 ml) was added to the mixture which yielded in an orange precipitate which was filtered, washed with EtOH and Et₂O and dried in vacuo. Yield: 25.4 g (91%).

### cis-Sodium bis(2,4-pentanedionato)dinitrocobaltate(III) (D).

To a stirred solution of **C** (2.0 g, 5.0 mmol) in H₂O (6.5 ml) was added a solution of NaOH (416 mg, 10.4 mmol) and acetylacetone (1.06 ml, 10.4 mmol) in H₂O (6.5 ml). The mixture was stirred for 5 min and filtered. The filtrate was allowed to stand overnight and the formed precipitate was filtered and washed with acetone and Et₂O. The crude product was dissolved in a minimum amount of H₂O and filtered into a saturated sodium nitrite solution of the same volume. A red precipitate was formed, which was filtered, washed with EtOH and Et₂O and dried in vacuo. Yield: 801 mg (44%). Anal.Calcd for C₁₀H₁₄CoN₂O₈Na (Mᵣ = 372.15 g/mol): C, 32.27; H, 3.79; N, 7.53. Found: C, 32.37; H, 3.76; N, 7.26. ¹H NMR (500.1 MHz, D₂O): δ 2.11 (s, 6H), 2.20 (s, 6H), 5.66 (s, 1H), 5.77 (s, 1H) ppm.

### Bis(2,4-pentanedionato) N⁶-(2-aminoethyl)- N⁴-(3-bromophenyl) quinazoline-4,6-diamine cobalt(III) hexafluorophosphate (1a).

Complex D (164 mg, 0.44 mmol) was dissolved in H₂O (2.5 ml) and MeOH (2.5 ml). **L₂** (200 mg, 0.46 mmol) in H₂O (1.7 ml) and MeOH (4 ml) was neutralized with NaOH (37 mg, 0.93 mmol) and added with activated charcoal (108 mg) to the cobalt complex solution. The resulting mixture was stirred for 20 min at room temperature, filtered through celite which was washed with small amounts of MeOH. NH₄PF₆ (400 mg, 2.5 mmol) in H₂O (500 µl) was added to the filtrate and the solution was stored at 0°C for 1 h and overnight at 4°C. Crude product was precipitated by the addition of H₂O (10 ml). The formed dark green residue was filtered off and washed with ice-cold H₂O and Et₂O. Further purification was performed using RP-HPLC (H₂O/MeOH, 5-95% MeOH). Yield: 78.8 mg (24%). Anal.Calcd for C₂₆H₃₀BrCoF₆N₅O₄P (Mᵣ = 760.35 g/mol): C, 41.07; H, 3.98; N, 9.21. Found: C, 40.76; H, 4.04; N, 9.07. ESI-MS in MeOH (positive): *m*/*z* 614, ([Co(acac)₂(**L**₂)]⁺); 514, ([Co(acac)(**L₂**) - H]⁺); 358, [(**L**₂)+H]⁺. ¹H NMR (500.1 MHz, DMSO-*d₆*): δ 1.63 (s, 3Hₐ, CH₃), 1.74 (s, 3H_{b}, CH₃), 1.96 (s, 3Hₐ, CH₃), 1.99 (s, 3Hₐ, CH₃ and s, 3H_{b}, CH₃), 2.09 (s, 3H_{b}, CH₃), 2.25 (s, 3Hₐ, 3H_{b}, CH₃), 2.67-2.83 (m, 2Hₐ, 2H_{b}), 2.84-2.98 (m, 1Hₐ, 1H_{b}), 3.59-3.68 (m, 1Hₐ), 3.69-3.79 (m, 1H_{b}), 4.77 (s, 1Hₐ, CH_{acac}), 5.54 (s, 1H_{b}, CH_{acac}), 5.56-5.64 (m, 1Hₐ, 1H_{b}, NH₂), 5.68 (s, 1Hₐ, CH_{acac}), 5.74 (s, 1H_{b}, CH_{acac}), 5.82-5.94 (m, 1Hₐ, 1H_{b}, NH₂), 7.28 (d, 1H_{b}), 7.35-7.44 (m, 2Hₐ, 2H_{b}), 7.49 (d, 1Hₐ), 7.66 (d, 1H_{b}), 7.70-7.76 (m, 1Hₐ, 1H_{b}), 7.77-7.84 (m, 1Hₐ, 1H_{b}), 8.04-8.17 (m, 3Hₐ, 1H_{b}), 8.61-8.76 (m, 1Hₐ, 2H_{b}), 9.89 (s, 1Hₐ), 10.04 (s, 1H_{b}) ppm. ¹³C NMR (125.75 MHz, DMSO-*d₆*): δ 26.2 (Cₐ), 26.4 (3Cₐ), 26.7 (2C_{b}), 26.9 (C_{b}), 27.1 (C_{b}), 42.4 (Cₐ), 42.5 (Cₐ), 51.7 (C_{b}), 52.7 (C_{b}), 96.4 (Cₐ), 98.2 (C_{b}), 98.3 (C_{b}), 98.5 (Cₐ), 113.8, 115.7 (Cₐ), 116.0, 121.7 121.8, 121.9, 129.4 (Cₐ), 125.3 (2C), 125.4, 127.0 (2C), 127.7 (2C), 130.7, 130.8, 131.1 (2C), 141.0, 141.2, 142.6, 143.2, 148.5, 157.4, 157.7, 189.1, 189.5, 189.6 (2C), 189.8 (2C), 190.3 (2C) ppm.

### Bis(2,4-pentanedionato) N⁶-(2-aminoethyl)- N⁴-(3-bromophenyl) quinazoline-4,6-diamine cobalt(III) chloride (1b).

Complex **D** (205.5 mg, 0.55 mmol) was dissolved in H₂O (4 ml) and MeOH (3 ml). **L₂** (250 mg, 0.58 mmol) in H₂O (2.5 ml) was neutralized with 0.23 M NaOH in MeOH (5.04 ml) and added with activated charcoal (135 mg) to the cobalt complex solution. The resulting mixture was stirred for 1 h at room temperature, filtered through celite which was washed with small amounts of MeOH. Brine (15 ml) was added to the filtrate and it was extracted with DCM (3 x 25 ml). The combined organic phases were dried over MgSO₄, filtered and concentrated under reduced pressure. Further purification was performed using RP-HPLC (H₂O/MeOH, 45-95% MeOH). Yield: 159.7 mg (44%). Anal.Calcd for C₂₆H₃₀BrClCoN₅O₄ · 0.5H₂O (Mᵣ = 659.85 g/mol): C, 47.33; H, 4.74; N, 10.61. Found: C, 47.37; H, 4.56; N, 10.49. ESI-MS in H₂O (positive): *m*/*z* 614, ([Co(acac)₂(**L₂**)]⁺); 514, ([Co(acac)(**L₂**) - H]⁺); 358, [(**L₂**)+H]⁺. ¹H NMR (500.1 MHz, DMSO-*d₆*): δ 1.63 (s, 3Hₐ, CH₃), 1.73 (s, 3H_{b}, CH₃), 1.96 (s, 3Hₐ, CH₃), 1.99 (s, 3Hₐ, CH₃ and s, 3H_{b}, CH₃), 2.09 (s, 3H_{b}, CH₃), 2.25 (s, 3Hₐ, 3H_{b}, 2·CH₃), 2.68-2.83 (m, 2Hₐ, 2H_{b}), 2.85-2.92 (m, 1Hₐ), 2.92-3.00 (m, 1H_{b}), 3.66-3.75 (m, 1Hₐ), 3.76-3.84 (m, 1H_{b}), 4.78 (s, 1Hₐ, CH_{acac}), 5.53 (s, 1H_{b}, CH_{acac}), 5.55-5.63 (m, 1Hₐ, 1H_{b}, NH₂), 5.67 (s, 1Hₐ, CH_{acac}), 5.73 (s, 1H_{b}, CH_{acac}), 5.80-5.90 (m, 1Hₐ, 1H_{b}, NH₂), 7.31 (dd, 1H_{b}), 7.35-7.43 (m, 2Hₐ, 2H_{b}), 7.49 (dd, 1Hₐ), 7.70 (d, 1Hₐ, 1H_{b}), 7.78 (d, 1H_{b}), 7.84-7.89 (m, 1Hₐ, 1H_{b}), 8.11 (dd, 1Hₐ), 8.12-8.17 (m, 1Hₐ, 1H_{b}), 8.23 (s, 1Hₐ), 8.64-8.70 (m, 1Hₐ, 2H_{b}), 10.06 (s, 1Hₐ), 10.16 (s, 1H_{b}) ppm.

Stability test revealed that compound **1a** and **1b** are stable for at least 24 h in minimal essential medium.

### Chemicals.

Erlotinib and all other investigated compounds were dissolved in DMSO. For experiments these stock solutions were further diluted into culture media at the indicated concentrations. The final DMSO concentrations were always less than 1%.

### Cell culture.

The human NSCLC cell lines A431 (wt EGFR-overexpressing and erlotinib-sensitive) and H1975 (EGFR mutation at L858R as well as T790M and erlotinib-resistant) were purchased from American Type Culture Collection (ATCC), Rockville, MD, USA and cultivated unless otherwise indicated in humidified incubators (37°C, 21% O₂, 5% CO₂) in RPMI 1640 medium containing 10% fetal calf serum (PAA, Linz Austria). Cell cultures were periodically checked for Mycoplasma contamination.

### Cytotoxicity assays.

Cells were plated (2x10³ cells/well) in 96-well plates and allowed to recover for 24 h. Subsequently, the dissolved drugs were added. After 72 h exposure, the proportion of viable cells was determined by MTT assay following the manufacturer's recommendations (EZ4U, Biomedica, Vienna, Austria). Cytotoxicity was expressed as IC₅₀ values calculated from full dose-response curves using Graph Pad Prism software.

### Hypoxic conditions.

Cells were plated in 96- or 6-well plates, respectively, in an HERA cell 150i (Thermo Scientific) with at 1% O₂/5% CO₂ level for indicated time points (72 h and 4 h for MTT assays and protein isolation, respectively) before analysis/harvesting.

### Western blot.

Proteins were isolated, resolved by SDS/PAGE, and transferred onto a polyvinylidenedifluoride membrane for Western blotting by routine methods. The following antibodies were used: EGFR, pEGFR (Tyr1068), ERK1/2 (p44/42 MAPK), and pERK (Thr202/Tyr204) (all polyclonal rabbit - Cell Signaling Technology, Beverly, MA, USA). β-actin was monoclonal mouse AC-15 (Sigma). All primary antibodies were used in 1:1000 dilutions. Additionally, horseradish peroxidase-labelled secondary antibodies from Santa Cruz Biotechnology were used at working dilutions of 1:10000.

### Animals.

Six- to 8-week-old female CB-17 scid/scid (SCID) mice were purchased from Harlan Laboratories (San Pietro al Natisone, Italy). The animals were kept in a pathogen-free environment and every procedure was done in a laminar airflow cabinet. The experiments were done according to the regulations of the Ethics Committee for the Care and Use of Laboratory Animals at the Medical University Vienna, The U.S. Public Health Service Policy on Human Care and Use of Laboratory Animals as well as the United Kingdom Coordinating Committee on Cancer Prevention Research's Guidelines for the Welfare of Animals in Experimental Neoplasia.

### Xenograft experiments.

For the local tumour growth experiments, A431 and Calu3 cells (1 × 10⁶) were injected subcutaneously into the right flank. Animals were randomly assigned to treatment groups and therapy was started when tumour nodules reached a mean size of 20 mm³. Animals were treated with erlotinib (orally 25 mg/kg dissolved in Cremophor EL diluted 1:1 in 96% ethanol and then diluted 1:10 with deionized water right before administration; five times a week for two weeks day 6-10 and 13-17) or **1b** (either intravenously 5 mg/kg on day 6, 8, 10, 15, 17, and 19, or intraperitoneally 25 mg/kg dissolved in 0.5% NaCl, five times a week for two weeks, day 6-10 and 13-17). Animals in the control group received the Cremophor EL solvent orally and PBS intraperitoneally. Animals were controlled for distress development every day and tumour size was assessed regularly by calliper measurement. Tumour volume was calculated using the formula: (length × width²)/2.

### Synthesis of EGFR inhibitors with a chelating moiety

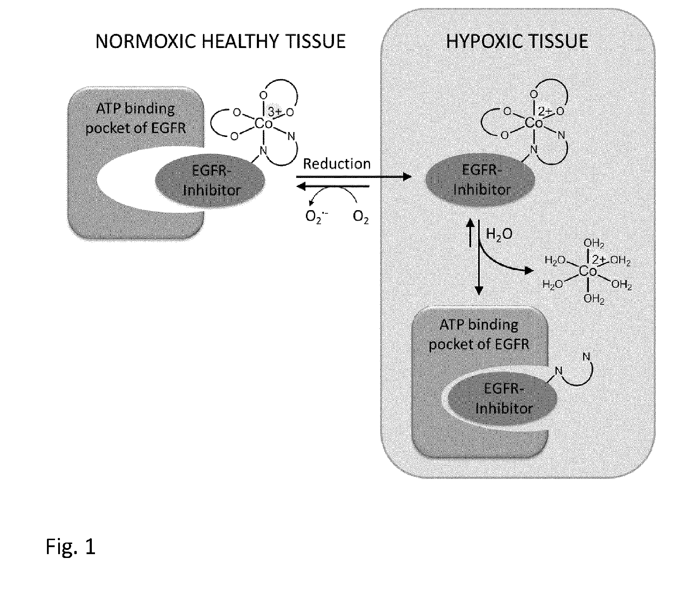

A schematic overview of the TKI Co(III)-prodrug concept is depicted in Figure 1. The core structure **A** was synthesized by a three-step procedure according to Tsou et al. (J Med Chem, 2001, 44, 2719-2734). **L1** was synthesized according to a modified literature procedure (Bourkoula et al., European Journal of Medicinal Chemistry, 2009, 44, 4021-4027). **L2** was obtained by direct reductive amination of *N*-Boc-2-aminoacetaldehyde with **A**, followed by deprotection using hydrochloric acid (Figure 2).

### EGFR-inhibitory activity of the ligands

In order to investigate whether the introduction of the chelating moiety interferes with their EGFR-inhibitory potential **L1** and **L2** were tested in comparison to the known EGFR inhibitor erlotinib. As a first step MTT assays (after 72 h treatment) performed in wild-type EGFR-overexpressing erlotinib-sensitive A431 and Calu3 cells as well as in H1975 cells, which harbour a resistance-conferring EGFR mutation T790M **(Table 1).** The 2-pyridinemethanimine moiety in **L1** distinctly reduced the anticancer activity, compared to erlotinib, especially in A431. Only in Calu3 cells activity in the low µM range was observed. In contrast, **L2** containing the smallest chelating moiety had IC₅₀ values in the low µM range in both erlotinib-responsive models. Moreover, also distinct activity against erlotinib-resistant H1975 was observed with this ethylenediamine-containing ligand.

**Table 1. Anticancer activity (IC₅₀ values)**

| | **Inhibition of Cell Growth (IC₅₀ µM)** | | |
|---|---|---|---|
| **Compound** | **A431** | **H1975** | **Calu3** |
| **erlotinib** | 2.57 ± 0.83 | >25 | 0.74± 0.02 |
| **L1** | > 25 | 20.4± 3.8 | 3.5± 0.08 |
| **L2** | 9.15 ± 0.47 | 15.5± 2.18 | 2.0± 0.52 |

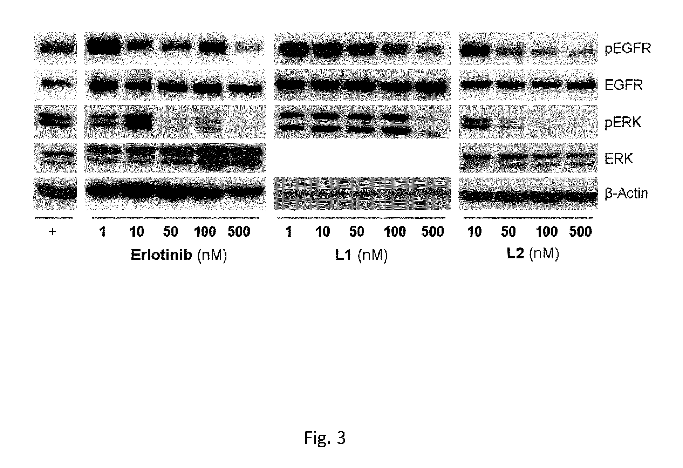

To verify that these effects are based on inhibition of the EGFR signaling pathway, the impact of the novel drugs on the ERK and EGFR phosphorylation levels of EGF-stimulated cells was analyzed by Western blotting (Figure 3). After 4 h drug incubation, all tested compounds revealed dose-dependent inhibitory effects, however, with differing potency. In accordance to the MTT data, **L2** was the most potent EGFR inhibitor with activity similar to erlotinib.

### Synthesis of Co(III) complexes

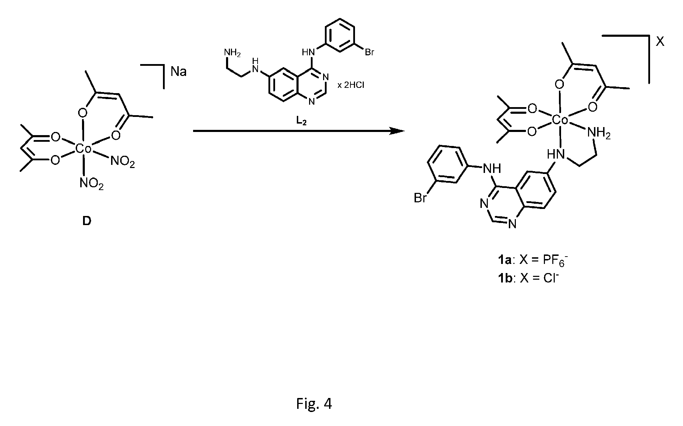

As Co(III) precursor for the complexation of **L2** Na[Co(acac)₂(NO₂)₂] **(D)** was used. The synthesis of **D** was performed in a two-step procedure. First hexanitrocobaltate(III) **(C)** was synthesized, which was further treated with an aqueous solution of acetylacetone forming the desired precursor **D** (Boucher et al., Journal of Inorganic & Nuclear Chemistry, 1965, 27, 1093-1098). Co(III) complexes of **L2** were prepared (similar to the method described by Ware et al. (J Med Chem, 1993, 36, 1839-1846)) in the presence of activated charcoal using a mixture of water and methanol as solvents (Figure 4).

### Biological testing of the cobalt complexes

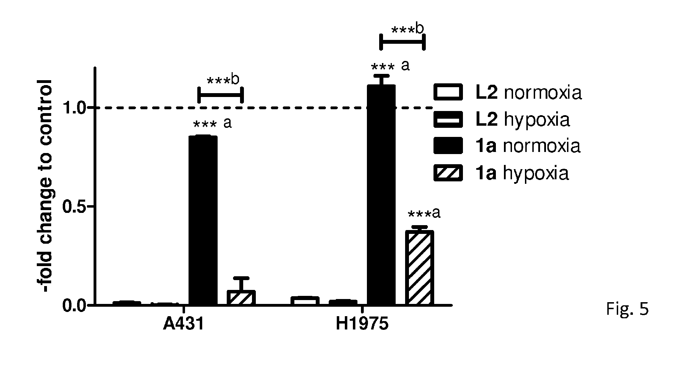

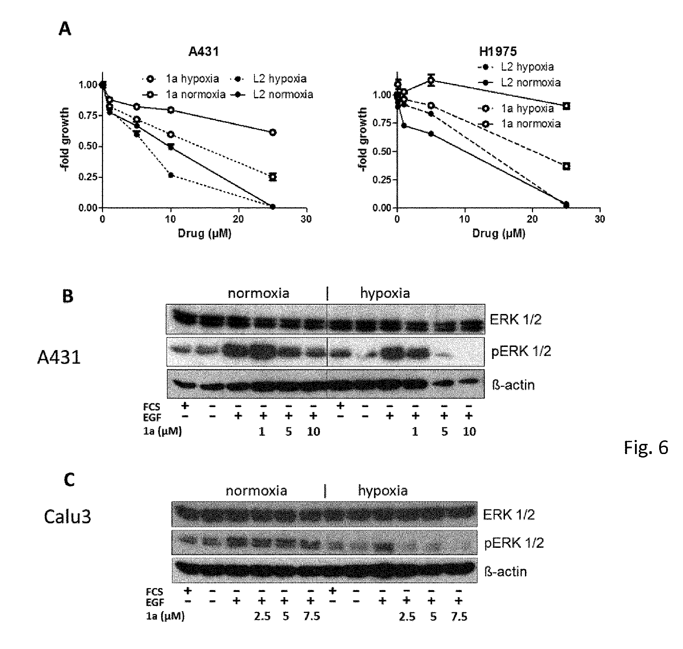

As a first step, the anticancer activity of the novel Co(III) complex was tested in comparison to the metal-free ligand under normoxic and hypoxic conditions as shown in Figures 5 and 6A after 72 h treatment cell viability was measured using a MTT-based system. Values given are means ± standard deviation (SD) of one representative experiment performed in triplicates (Statistical analysis: t-test; ***p< 0.001 ^{a}: difference between **L2** and **1a**; ^{b}: difference between **1a** normoxia and **1a** hypoxia). **1a** (up to the highest tested concentration of 25 µM) was widely inactive under normoxic conditions, in contrast, when cells were treated under hypoxia the activity of **1a** was significantly increased.

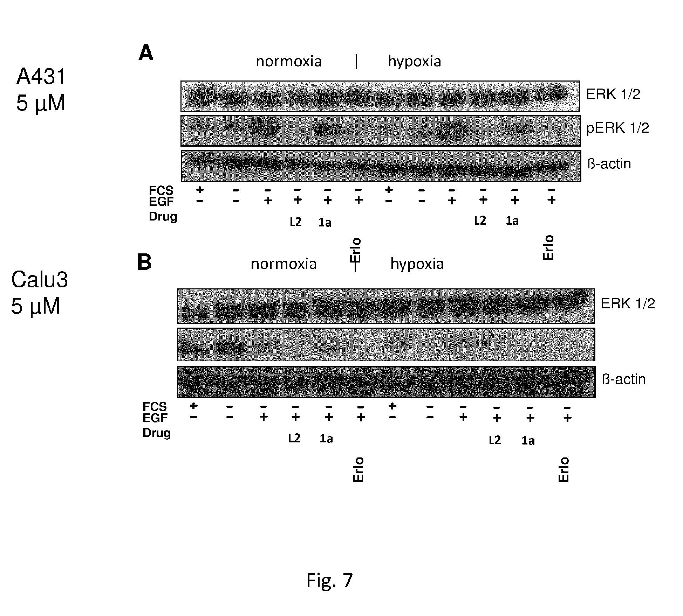

In accordance, subsequent Western blot analysis of A431 and Calu3 cells (grown in medium with (+) or without (-) FCS, treated for 4 h with the indicated drug concentration of **1a** (after EGFR stimulation with 50 ng/ml medium EGF for 15 min, cells were harvested) revealed that also the EGFR inhibitory potential of **1a** was distinctly reduced under normoxic conditions (Figures 6B, C and 7A, B), while potent activity similar to the metal-free ligand L2 was found under hypoxia (Figures 7A). Together, this shows hypoxia-specific activation of the novel Co(III) complex in cell culture experiments.

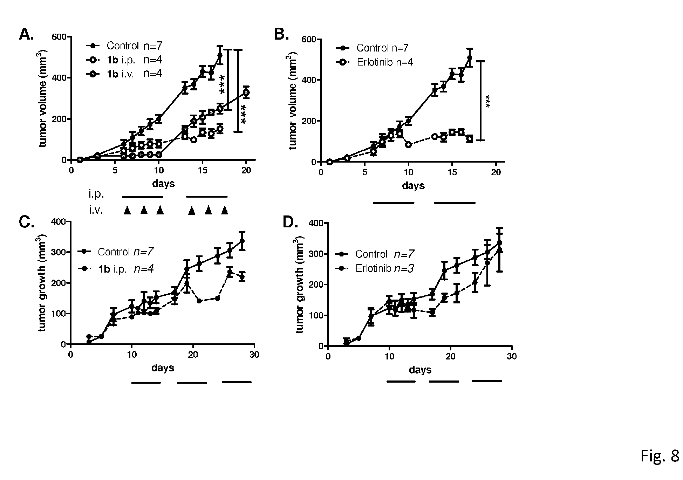

As a next step the activity of the new drug was tested against EGFR-driven xenografts in SCID mice (Figure 8). In general, **1b** treatment was well tolerated even after repeated applications (5 mg/kg for i.v. and 25 mg/kg for i.p. application; therapy days are indicated by arrows and black bars).

With regard to its anticancer activity, **1b** potently inhibited tumor growth of A431 xenografts with an efficacy comparable to erlotinib (especially upon intraperitoneal application) (Figure 8A, B). Notably, in Calu3 xenografts **1b** induced distinct tumor regression when a tumour size of more than 200 mm² was reached (Figure 8C, D). This is of interest, as Calu3 xenografts are characterized by a rather specific tumor histology, where small tumour islands are surrounded by murine fibroblasts. Consequently, in these xenografts a larger tumor volume might be necessary for the development of hypoxic conditions and, thus, activation of **1b**.

The present invention discloses the following preferred embodiments:
1. Compound comprising a Co³⁺ ion and a tyrosine kinase inhibitor moiety, wherein the tyrosine kinase inhibitor moiety comprises a Co³⁺-chelating moiety, and wherein the compound is bis(2,4-pentanedionato) N⁶-(2-aminoethyl)- N⁴-(3-bromophenyl)quinazoline-4,6-diamine cobalt(III) hexafluorophosphate or bis(2,4-pentanedionato) N⁶-(2-aminoethyl)-N⁴-(3-bromophenyl) quinazoline-4,6-diamine cobalt(III) chloride, according to the following Formula (A): 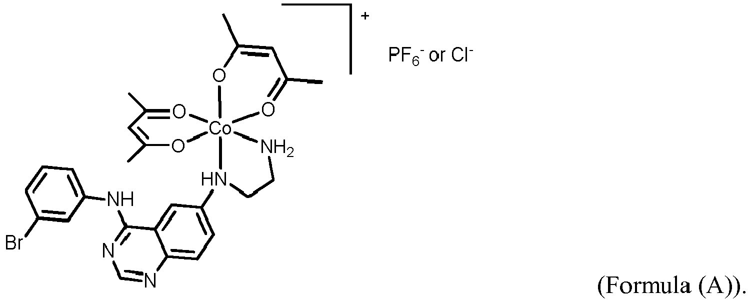
2. Compound comprising a Co³⁺ ion and a tyrosine kinase inhibitor moiety, wherein the tyrosine kinase inhibitor moiety comprises a Co³⁺-chelating moiety.
3. Compound according to embodiment 1 or 2, wherein the tyrosine kinase inhibitor moiety is a 4-anilinoquinazoline tyrosine kinase inhibitor moiety.
4. Compound according to embodiment 2 or embodiment 3, wherein the 4-anilinoquinazoline tyrosine kinase inhibitor moiety has the general formula (B), which includes a Co³⁺-chelating moiety 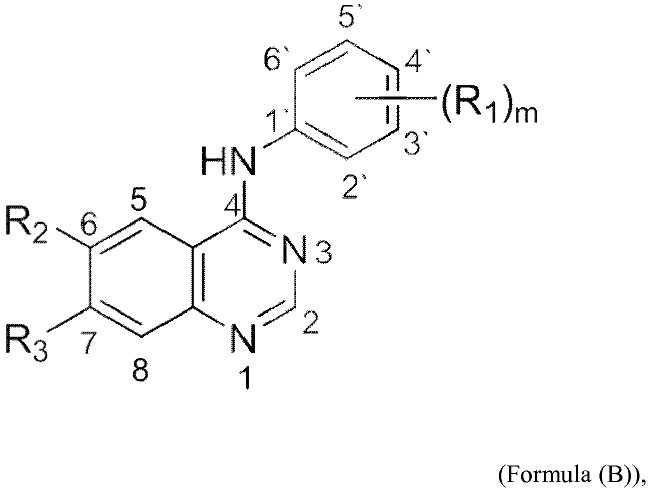 wherein m is 0,1,2, or 3
   each R₁ is independently selected from the group consisting of amino, hydrogen, halogen, hydroxy, nitro, carboxy, formyl, cyano, trifluoromethyl, trifluoromethoxy, carbamoyl, ureido, guanidino, C₁₋₈ alkyl, C₂₋₈ alkenyl, C₂₋₈ alkynyl, C₁₋₈ alkoxy, C₃₋₈ cycloalkoxyl, C₁₋₈ alkylcarbonyl, phenyl, phenoxy, piperidino, morpholino, thiomorpholino or 2-fluorobenzyloxy, 3-fluorobenzyloxy, 4-fluorobenzyloxy;
   and
   R₂ and R₃ together form a Co³⁺-chelating moiety, or
   R₂ and R₃ are individually selected from the group consisting of: a chelating moiety or hydrogen, alkyl, alkoxyalkyl,alkylthio, cycloalkyl, hydroxy, alkoxy, alkoxyalkyl, aralkoxy, aryl, halo, haloalkyl, carboxy,carbalkoxy and Formula (C), 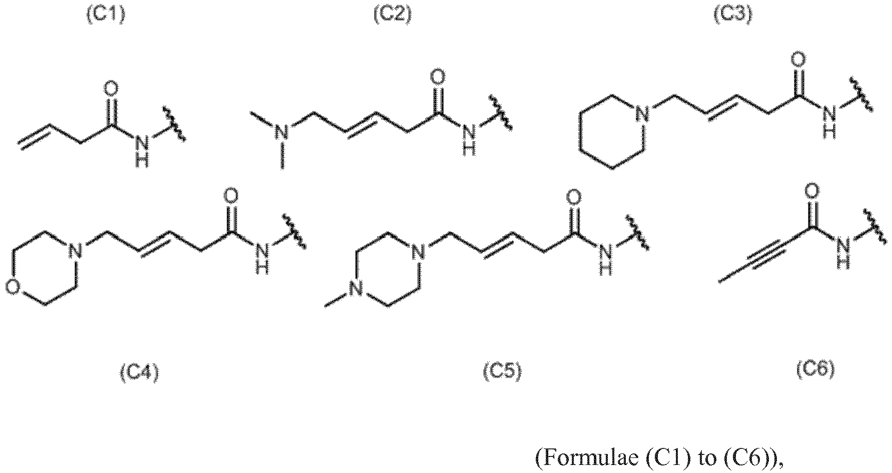 with the proviso that at least one of R₂ or R₃ is a Co³⁺-chelating moiety or R₂ and R₃ together form a Co³⁺-chelating moiety.
5. Compound according to any one of embodiments 2 to 4, wherein the Co³⁺-chelating moiety is bidentate.
6. Compound according to any one of embodiments 2 to 5, wherein the Co³⁺-chelating moiety is selected from the following Formulae (D1) to (D6), 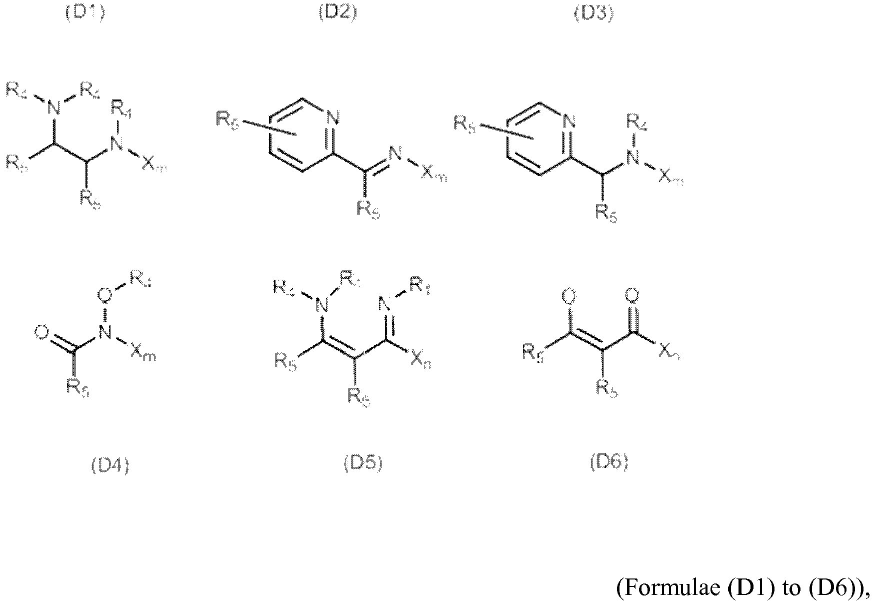 wherein m is 0 or 1 and n is 1
   R₄ is independently selected from hydrogen or C₁₋₈ alkyl;
   R₅ is independently selected from hydrogen, halogen, hydroxy, nitro, carboxy, formyl, cyano, trifluoromethyl, trifluoromethoxy, carbamoyl, ureido, guanidino, C₁₋₈alkyl,alkenyl, alkynyl, alkylthio, cycloalkyl, alkoxy, alkoxyalkyl, aralkoxy,aryl, haloalkyl, a phenyl group or a 5- or 6-membered heterocyclic ring containing 1 to 4 heteroatoms selected from N, O, or S; and
   wherein X is C₁₋₈ alkyl, carbamoyl-C₁₋₈ alkyl, N-C₁₋₈ alkylcarbamoyl-C₁₋₈alkyl, N-C₁₋₈ alkylcarbamoyl or CH₂CH₂O(CH₂CH₂O)_{z}CH₂CH₂ (with z = 0-5).
7. Compound according to any one of embodiments 2 to 6, wherein the TKI moiety is N⁶-(2-aminoethyl)-N⁴-(3-bromophenyl)quinazoline-4,6-diamine dihydrochloride.
8. Compound according to any one of embodiments 2 to 7, wherein the compound is bis(2,4-pentanedionato) N⁶-(2-aminoethyl)- N⁴-(3-bromophenyl)quinazoline-4,6-diamine cobalt(III) chloride or bis(2,4-pentanedionato) N⁶-(2-aminoethyl)- N⁴-(3-bromophenyl)quinazoline-4,6-diamine cobalt(III) hexafluorophosphate.
9. Compound according to any one of embodiments 2 to 8, comprising a further component, namely one or two mono-,bi- or tetradentate ligand(s).
10. Compound according to any one of embodiments 2 to 9, comprising a further component, which is a ligand independently selected from the group consisting of: Formulae (E1) to (E5): 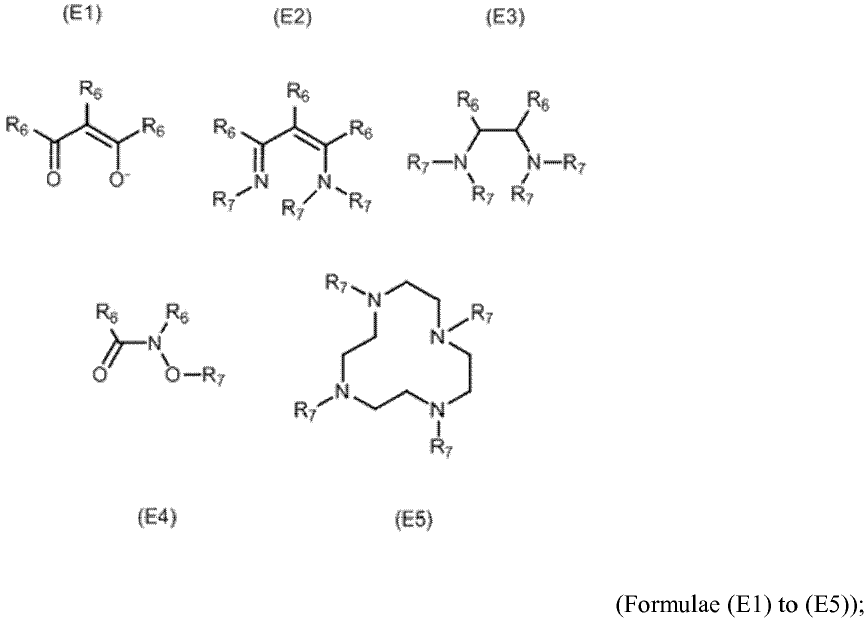 halido, nitrite, and ammine
   wherein R₆ is independently selected from the group consisting of: hydrogen, halogen, hydroxy, nitro, carboxy, formyl, cyano, trifluoromethyl, trifluoromethoxy, carbamoyl, ureido, guanidino, alkyl, alkenyl, alkynyl, alkylthio, cycloalkyl, alkoxy, alkoxyalkyl, aralkoxy, aryl, and haloalkyl;
   and R₇ is independently selected from the group consisting of hydrogen and C₁₋₈ alkyl.
11. Compound according to any one of embodiments 2 to 10, comprising a counter ion Yₙ (with n is 0-3), which is independently selected from chloride, bromide, carbonate, RCOO⁻ (with R = C₁₋₆ alkyl, C₁₋₆ alkenyl), sulfate, phosphate, methanesulphonate, trifluorosulphonate, benzenesulfonate, p-toluene sulphonate, 1-napthalene sulfonate, acetate, trifluoroacetate, malate, tartrate, citrate, lactate, oxalate, salicylate, hydrogen, sodium, potassium, ammonium, phosphonium, pyrrolidinium, pyridinium, and imidazolium.
12. Compound according to any one of embodiments 1 to 11, for use in a therapeutic treatment.
13. Compound according to any one of embodiments 1 to 11 for use in the treatment of a hyperproliferative disorder in a mammal which comprises administering to said mammal a pharmaceutically effective amount of the compound of any one of embodiments 1 to 11.
14. Compound according to any one of embodiments 1 to 11 for use in the treatment of carcinomas, myeloid disorders or adenomas.
15. Compound according to any one of embodiments 1 to 11, for use in the treatment of cancer, preferably non-small cell lung cancer (NSCLC), head and neck cancer, colorectal cancer, prostate cancer, bladder cancer, esophageal cancer, pancreatic cancer, gastroesophageal cancer, ovarian cancer, cervical cancer, astrocytoma grade 1-4, renal cell cancer, and prostate cancer.
16. Compound according to any one of embodiments 1 to 11 for use in producing a receptor tyrosine kinase inhibitory effect in humans or warm-blooded animals in need of such treatment which comprises administering to said animal an effective amount.
17. Compound according to any one of embodiments 1 to 11 for use in treating in humans or a warm-blooded animal a disease or medical condition mediated alone or in part by the enzyme receptor tyrosine kinase.
18. A pharmaceutical formulation comprising at least one compound of embodiment 1 to 11 or a pharmaceutically acceptable salt thereof, together with one or more pharmaceutically acceptable carriers, diluents or excipients.
19. A method for treating a cancer patient comprising:
   (i) identification of EGFR gene mutations in an overproliferating cell type or tumour extracted from the patient; and
   (ii) administering a compound according to any one of embodiments 1 to 11 to the patient.

## Claims

1. Compound comprising a Co³⁺ ion and a tyrosine kinase inhibitor (TKI) moiety, wherein the TKI moiety comprises a Co³⁺-chelating moiety, and wherein the compound is bis(2,4-pentanedionato) N⁶-(2-aminoethyl)- N⁴-(3-bromophenyl)quinazoline-4,6-diamine cobalt(III) hexafluorophosphate or bis(2,4-pentanedionato) N⁶-(2-aminoethyl)-N⁴-(3-bromophenyl) quinazoline-4,6-diamine cobalt(III) chloride, according to the following Formula (A):

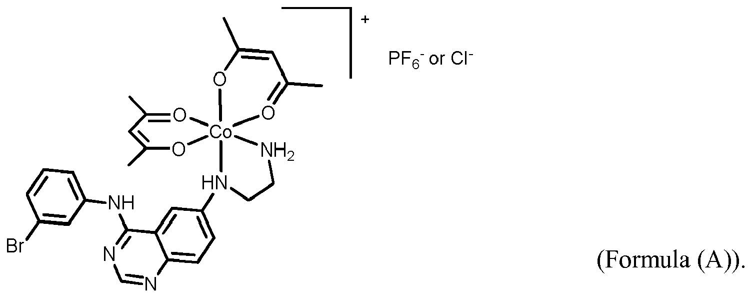

2. Compound comprising a Co³⁺ ion and a TKI moiety, wherein the TKI moiety comprises a Co³⁺-chelating moiety.

3. Compound according to claim 1 or 2, wherein the TKI moiety is a 4-anilinoquinazoline TKI moiety.

4. Compound according to claim 2 or claim 3, wherein the 4-anilinoquinazo line TKI moiety has the general formula (B), which includes a Co³⁺-chelating moiety 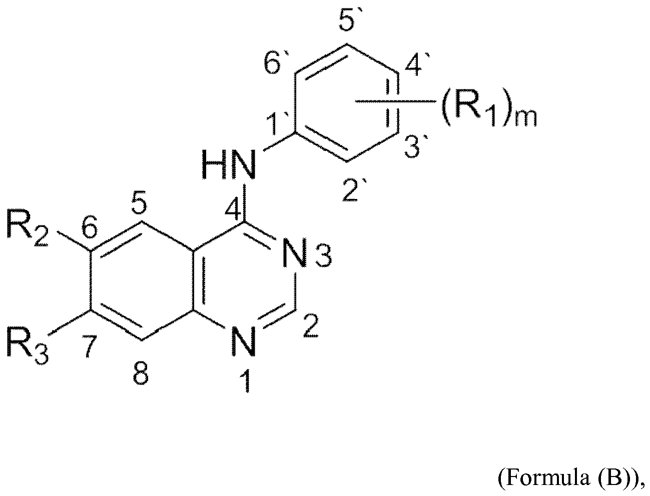 wherein m is 0,1,2, or 3
each R₁ is independently selected from the group consisting of amino, hydrogen, halogen, hydroxy, nitro, carboxy, formyl, cyano, trifluoromethyl, trifluoromethoxy, carbamoyl, ureido, guanidino, C₁₋₈ alkyl, C₂₋₈ alkenyl, C₂₋₈ alkynyl, C₁₋₈ alkoxy, C₃₋₈ cycloalkoxyl, C₁₋₈ alkylcarbonyl, phenyl, phenoxy, piperidino, morpholino, thiomorpholino or 2-fluorobenzyloxy, 3-fluorobenzyloxy, 4-fluorobenzyloxy;
and
R₂ and R₃ together form a Co³⁺-chelating moiety, or
R₂ and R₃ are individually selected from the group consisting of: a chelating moiety or hydrogen, alkyl, alkoxyalkyl, alkylthio, cycloalkyl, hydroxy, alkoxy, alkoxyalkyl, aralkoxy, aryl, halo, haloalkyl, carboxy, carbalkoxy and Formula (C), 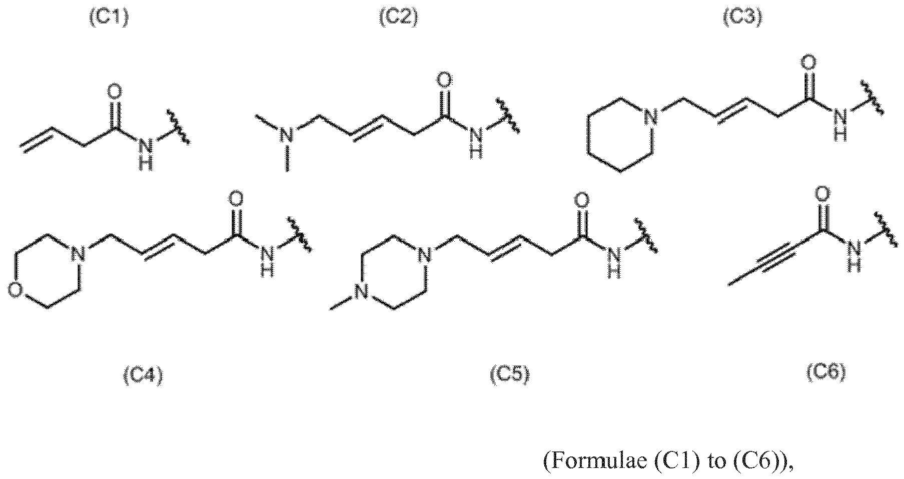 with the proviso that at least one of R₂ or R₃ is a Co³⁺-chelating moiety or R₂ and R₃ together form a Co³⁺-chelating moiety.

5. Compound according to any one of claims 2 to 4, wherein the Co³⁺-chelating moiety is bidentate.

6. Compound according to any one of claims 2 to 5, wherein the Co³⁺-chelating moiety is selected from the following Formulae (D1) to (D6), 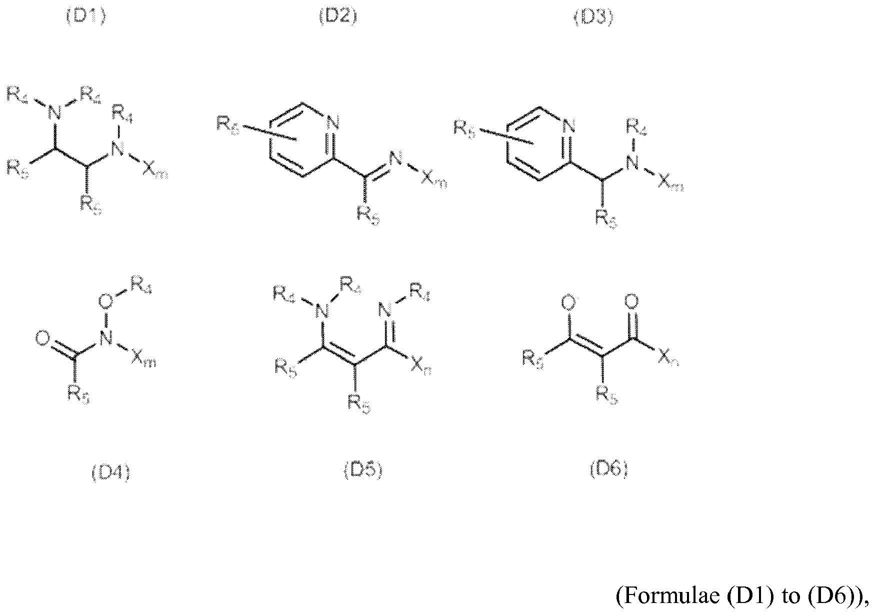 wherein m is 0 or 1 and n is 1
R₄ is independently selected from hydrogen or C₁₋₈ alkyl;
R₅ is independently selected from hydrogen, halogen, hydroxy, nitro, carboxy, formyl, cyano, trifluoromethyl, trifluoromethoxy, carbamoyl, ureido, guanidino, C₁₋₈alkyl,alkenyl, alkynyl, alkylthio, cycloalkyl, alkoxy, alkoxyalkyl, aralkoxy, aryl, haloalkyl, a phenyl group or a 5- or 6-membered heterocyclic ring containing 1 to 4 heteroatoms selected from N, O, or S; and
wherein X is C₁₋₈alkyl, carbamoyl-C₁₋₈ alkyl, N-C₁₋₈ alkylcarbamoyl-C₁₋₈alkyl, N-C₁₋₈ alkylcarbamoyl or CH₂CH₂O(CH₂CH₂O)_{z}CH₂CH₂ (with z = 0-5).

7. Compound according to any one of claims 2 to 6, wherein the tyrosine kinase inhibitor moiety is N⁶-(2-aminoethyl)-N⁴-(3-bromophenyl)quinazoline-4,6-diamine dihydrochloride.

8. Compound according to any one of claims 2 to 7, wherein the compound is bis(2,4-pentanedionato) N⁶-(2-aminoethyl)- N⁴-(3-bromophenyl)quinazoline-4,6-diamine cobalt(III) chloride or bis(2,4-pentanedionato) N⁶-(2-aminoethyl)- N⁴-(3-bromophenyl)quinazoline-4,6-diamine cobalt(III) hexafluorophosphate.

9. Compound according to any one of claims 2 to 8, comprising a further component, namely one or two mono-,bi- or tetradentate ligand(s).

10. Compound according to any one of claims 2 to 9, comprising a further component, which is a ligand independently selected from the group consisting of: Formulae (E1) to (E5): 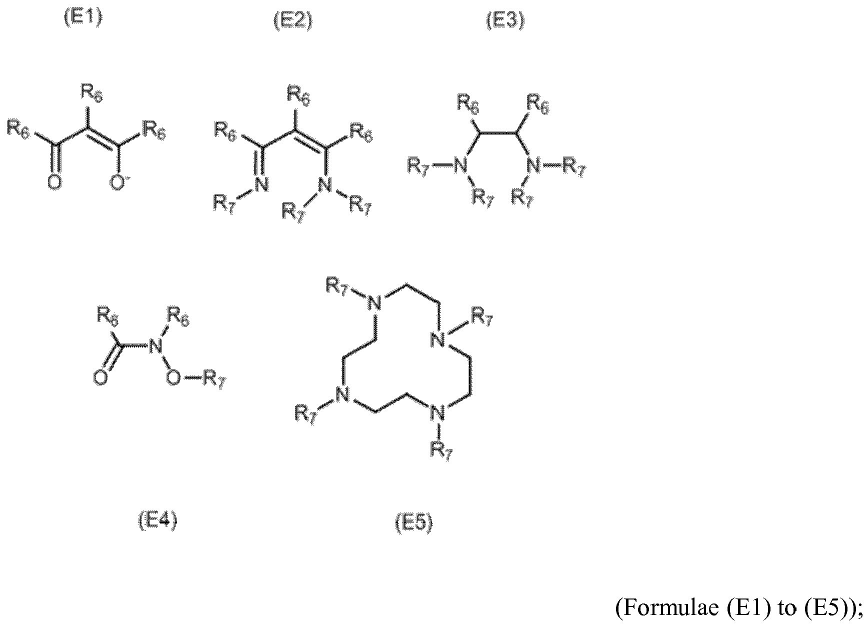 halido, nitrite, and ammine
wherein R₆ is independently selected from the group consisting of : hydrogen, halogen, hydroxy, nitro, carboxy, formyl, cyano, trifluoromethyl, trifluoromethoxy, carbamoyl, ureido, guanidino, alkyl, alkenyl, alkynyl, alkylthio, cycloalkyl, alkoxy, alkoxyalkyl, aralkoxy, aryl, and haloalkyl;
and R₇ is independently selected from the group consisting of hydrogen and C₁₋₈alkyl.

11. Compound according to any one of claims 2 to 10, comprising a counter ion Yₙ (with n is 0-3), which is independently selected from chloride, bromide, carbonate, RCOO⁻ (with R = C₁₋₆alkyl, C₁₋₆alkenyl), sulfate, phosphate, methanesulphonate, trifluorosulphonate, benzenesulfonate, p-toluene sulphonate, 1-napthalene sulfonate, acetate, trifluoroacetate, malate, tartrate, citrate, lactate, oxalate, salicylate, hydrogen, sodium, potassium, ammonium, phosphonium, pyrrolidinium, pyridinium, and imidazolium.

12. Compound according to any one of claims 1 to 11, for use in a therapeutic treatment.

13. Compound according to any one of claims 1 to 11 for use in the treatment of a hyperproliferative disorder in a mammal which comprises administering to said mammal a pharmaceutically effective amount of the compound of any one of claims 1 to 11.

14. Compound according to any one of claims 1 to 11 for use in the treatment of carcinomas, myeloid disorders or adenomas, cancer, preferably non-small cell lung cancer (NSCLC), head and neck cancer, colorectal cancer, prostate cancer, bladder cancer, esophageal cancer, pancreatic cancer, gastroesophageal cancer, ovarian cancer, cervical cancer, astrocytoma grade 1-4, renal cell cancer and prostate cancer; or for use in producing a receptor tyrosine kinase inhibitory effect in humans or warm-blooded animals in need of such treatment which comprises administering to said animal an effective amount.

15. A pharmaceutical formulation comprising at least one compound of claim 1 to 11 or a pharmaceutically acceptable salt thereof, together with one or more pharmaceutically acceptable carriers, diluents or excipients.
